# EUROPEAN PATENT SPECIFICATION

(11) **EP 4 073 044 B1**
(45) Date of publication and mention of the grant of the patent: **19.02.2025**
(21) Application number: 20817376.5
(22) Date of filing: 08.12.2020
(51) Int. Cl.: C07D 239/90, C07D 403/12, A61P 35/00, A61K 31/517

(54) **NEW BRAF INHIBITORS AS PARADOX BREAKERS**
NEUE BRAF-INHIBITOREN ALS PARADOX-BRECHER
NOUVEAUX INHIBITEURS DE BRAF EN TANT QUE PARADOX BREAKERS

(30) Priority: 10.12.2019 EP 19214867
(43) Date of publication of application: 19.10.2022
(73) Proprietor: F. Hoffmann-La Roche AG, 4070 Basel (CH)
(72) Inventor: DOLENTE, Cosimo, 4070 Basel (CH); HEWINGS, David Stephen, 4070 Basel (CH); HUNZIKER, Daniel, 4070 Basel (CH); KRUMMENACHER, Daniela, 4070 Basel (CH); PETTAZZONI, Piergiorgio Francesco Tommaso, 4070 Basel (CH); WICHMANN, Juergen, 4070 Basel (CH)
(74) Representative: Vitra, Hermeto
(86) International application number: PCT/EP2020/084969
(87) International publication number: WO 2021/116050

(56) References cited:
- WO-A1-2012/118492
- WO-A1-2020/261156
- WENGLOWSKY STEVE ET AL: "Highly potent and selective 3-N-methylquinazoline-4(3H)-one based inhibitors of B-RafV600Ekinase", BIOORGANIC & MEDICINAL CHEMISTRY LETTERS, PERGAMON, AMSTERDAM, NL, vol. 24, no. 8, 13 March 2014 (2014-03-13), pages 1923 - 1927, XP028841756, ISSN: 0960-894X, DOI: 10.1016/J.BMCL.2014.03.007

## Description

The present invention provides a compound of formula (I) which is a BRAF inhibitor and does have paradox breaking properties, its manufacture, pharmaceutical compositions containing it and its use as therapeutically active substance.

The present invention provides a novel compound of formula (I) 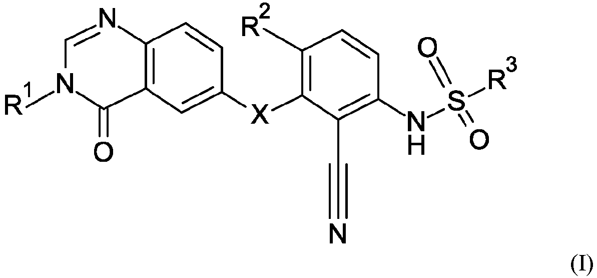
R¹ is C₁₋₆-alkyl;
X is selected from
   i) -NH-, and
   ii) -O-;
R² is selected from
   iii) H,
   iv) cyano, and
   v) halogen;
R³ is selected from
   vi) NR⁴R⁵, and
   vii) CHR⁶R⁷;
R⁴ is selected from
   viii) C₁₋₆-alkyl,
HV / 11.11.2020
   ix) C₃₋₈-cycloalkyl, and
   x) C₃₋₈-cycloalkyl-C₁₋₆-alkyl;
R⁵ is selected from
   xi) C₁₋₆-alkyl,
   xii) C₃₋₈-cycloalkyl, and
   xiii) C₃₋₈-cycloalkyl-C₁₋₆-alkyl;
or R⁴ and R⁵ together with the nitrogen atom to which they are attached form an heterocycloalkyl optionally substituted with R⁸, wherein the heterocycloalkyl is selected from pyrrolidinyl and piperidinyl;
R⁶ is selected from
   xiv) C₁₋₆-alkyl,
   xv) C₃₋₈-cycloalkyl, and
   xvi) C₃₋₈-cycloalkyl-C₁₋₆-alkyl;
R⁷ is selected from
   xvii) C₁₋₆-alkyl,
   xviii) C₃₋₈-cycloalkyl, and
   xix) C₃₋₈-cycloalkyl-C₁₋₆-alkyl;
or R⁶ and R⁷ together with the carbon atom to which they are attached form a C₃₋₈-cycloalkyl optionally substituted with R⁸; and
R⁸ is halogen;
with the proviso that N-[2-cyano-4-fluoro-3-(3-methyl-4-oxo-quinazolin-6-yl)oxy-phenyl]-3-fluoro-pyrrolidine-1-sulfonamide is excluded
or a pharmaceutically acceptable salt thereof.

The Rapidly Accelerated Fibrosarcoma (RAF) class of serine-threonine kinases comprise three members (ARAF, BRAF, RAF1) that compose the first node of the MAP kinase signalling pathway. Despite the apparent redundancy of the three RAF isoforms in signalling propagation through phosphorylation of MEK1 and 2, frequent oncogenic activating mutations are commonly found only for BRAF. In particular, substitution of V600 with glutamic acid or lysine renders the kinase highly activated with consequent hyper-stimulation of the MAPK pathway, independently from external stimulations (Cell. 2015 Jun 18; 161(7): 1681-1696.)

Mutant BRAF is a targetable oncogenic driver and three BRAF inhibitors (vemurafenib, dabrafenib and encorafenib) reached the market up to now showing efficacy in BRAFV600E-positive melanoma. However rapid acquisition of drug resistance is almost universally observed and the duration of the therapeutic benefits for the targeted therapy remains limited.

Moreover, the developed BRAF inhibitors revealed an unexpected and "paradoxical" ability to repress MAPK signalling in BRAFV600E-driven tumours while the same inhibitors presented MAPK stimulatory activities in BRAF wild type (WT) models (N Engl J Med 2012; 366:271-273; and British Journal of Cancer volume 111, pages640-645(2014)).

Mechanistic studies on the RAF paradox then clarified that oncogenic BRAFV600E phosphorylates MEK 1/2 in its monomeric cytosolic form while WT BRAF and RAF1 activation requires a complex step of events including cell membrane translocation and homo and/or heterodimerization promoted by activated RAS (KRAS, NRAS, HRAS) (Nature Reviews Cancer volume 14, pages455-467(2014)).

The binding of inhibitors like vemurafenib, dabrafenib or encorafenib to a WT BRAF or RAF1 protomer, quickly induces RAF homo and/or hetero dimerization and membrane association of the newly formed RAF dimer. In the dimeric conformation, one RAF protomer allosterically induces conformational changes of the second resulting in a kinase active status and, importantly, in a conformation unfavourable for the binding of the inhibitor. The dimer induced by drug treatment, as a result, promotes MEK phosphorylation by the catalysis operated by the unbound protomer with hyperactivation of the pathway.

The RAF paradox results in two clinically relevant consequences: 1) accelerated growth of secondary tumours upon BRAFi monotherapy (mainly keratochantoma and squamous-cell carcinomas) (N Engl J Med 2012; 366:271-273) and 2) the acquisition of drug resistance in the setting of BRAFi monotherapy as well as in combinations of BRAFi+MEKi presents activation of dimer-mediated RAF signalling by genetically driven events including RAS mutations, BRAF amplifications, expression of dimeric-acting BRAF splice variants (Nature Reviews Cancer volume 14, pages455-467(2014)).

The present invention relates to the surprising finding that the BRAF inhibitor of formula (I) shows considerably less paradoxial activation of the MAPK signalling pathway while retaining high potency. This compound can also be referred to as a paradox breaker or RAF paradox breaker, compared to compounds inducing the RAF paradox (and which could be referred to as paradox inducers or RAF paradox inducers).

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like.

The term "protecting group" (PG) denotes a group which selectively blocks a reactive site in a multifunctional compound such that a chemical reaction can be carried out selectively at another unprotected reactive site in the meaning conventionally associated with it in synthetic chemistry. Protecting groups can be removed at the appropriate point. Exemplary protecting groups are amino-protecting groups, carboxy-protecting groups or hydroxy-protecting groups. Particular protecting groups are the tert-butoxycarbonyl (Boc), benzyloxycarbonyl (Cbz), fluorenylmethoxycarbonyl (Fmoc) and benzyl (Bn) groups. Further particular protecting groups are the tert-butoxycarbonyl (Boc) and the fluorenylmethoxycarbonyl (Fmoc) groups. More particular protecting group is the tert-butoxycarbonyl (Boc) group.

The abbreviation uM means microMolar and is equivalent to the symbol µM.

The abbreviation uL means microliter and is equivalent to the symbol µL.

The abbreviation ug means microgram and is equivalent to the symbol µg.

The compound of formula (I) can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention is the compound according to formula (I) as described herein and pharmaceutically acceptable salts, in particular the compound according to formula (I) as described herein.

A particular embodiment of the present invention provides a compound according to formula (I) as described herein, wherein
R¹ is C₁₋₆-alkyl;
X is selected from
   i) -NH-, and
   i) -O-;
R² is selected from
   ii) H, and
   iii) halogen;
R³ is selected from
   iv) NR⁴R⁵, and
   v) CHR⁶R⁷;
R⁴ is selected from
   vi) C₁₋₆-alkyl,
   vii) C₃₋₈-cycloalkyl, and
   viii) C₃₋₈-cycloalkyl-C₁₋₆-alkyl;
R⁵ is selected from
   ix) C₁₋₆-alkyl,
   x) C₃₋₈-cycloalkyl, and
   xi) C₃₋₈-cycloalkyl-C₁₋₆-alkyl;
or R⁴ and R⁵ together with the nitrogen atom to which they are attached form an heterocycloalkyl optionally substituted with R⁸, wherein the heterocycloalkyl is selected from pyrrolidinyl and piperidinyl;
R⁶ is selected from
   xii) C₁₋₆-alkyl,
   xiii) C₃₋₈-cycloalkyl, and
   xiv) C₃₋₈-cycloalkyl-C₁₋₆-alkyl;
R⁷ is selected from
   xv) C₁₋₆-alkyl,
   xvi) C₃₋₈-cycloalkyl, and
   xvii) C₃₋₈-cycloalkyl-C₁₋₆-alkyl;
or R⁶ and R⁷ together with the carbon atom to which they are attached form a cyclopentyl or a cyclohexyl ring optionally substituted with R⁸;
R⁸ is halogen;
with the proviso that (3R)-N-[2-cyano-4-fluoro-3-(3-methyl-4-oxo-quinazolin-6-yl)oxyphenyl]-3-fluoro-pyrrolidine-1-sulfonamide is excluded
or a pharmaceutically acceptable salt thereof.

A particular embodiment of the present invention provides a compound according to formula (I) as described herein, wherein
R¹ is methyl;
X is selected from
   i) -NH-, and
   ii) -O-;
R² is selected from
   iii) H,
   iv) chloro, and
   v) fluoro;
R³ is selected from
   vi) NR⁴R⁵, and
   vii) CHR⁶R⁷;
R⁴ is selected from
   viii) methyl,
   ix) ethyl,
   x) propyl,
   xi) cyclopropyl, and
   xii) cyclopropylmethyl;
R⁵ is selected from
   xiii) methyl,
   xiv) ethyl,
   xv) propyl,
   xvi) cyclopropyl, and
   xvii) cyclopropylmethyl;
or R⁴ and R⁵ together with the nitrogen atom to which they are attached form an heterocycloalkyl optionally substituted with R⁸, wherein the heterocycloalkyl is selected from pyrrolidinyl and piperidinyl;
R⁶ is selected from
   xviii) methyl,
   xix) ethyl,
   xx) propyl,
   xxi) cyclopropyl, and
   xxii) cyclopropylmethyl;
R⁷ is selected from
   xxiii) methyl,
   xxiv) ethyl,
   xxv) propyl,
   xxvi) cyclopropyl, and
   xxvii) cyclopropylmethyl;
or R⁶ and R⁷ together with the carbon atom to which they are attached form a cyclopentyl or a cyclohexyl ring optionally substituted with R⁸;
R⁸ is fluoro;
with the proviso that (3R)-N-[2-cyano-4-fluoro-3-(3-methyl-4-oxo-quinazolin-6-yl)oxyphenyl]-3-fluoro-pyrrolidine-1-sulfonamide is excluded
or a pharmaceutically acceptable salt thereof.

A particular embodiment of the present invention provides a compound according to formula (I) as described herein, wherein
R¹ is methyl;
X is selected from
   i) -NH-, and
   ii) -O-;
R² is selected from
   iii) H,
   iv) chloro, and
   v) fluoro;
R³ is selected from
   vi) NR⁴R⁵, and
   vii) CHR⁶R⁷;
R⁴ is methyl;
R⁵ is ethyl;
or R⁴ and R⁵ together with the nitrogen atom to which they are attached form a pyrrolidinyl ring optionally substituted with R⁸;
R⁶ is methyl;
R⁷ is ethyl;
or R⁶ and R⁷ together with the nitrogen atom to which they are attached form a cyclopentyl or a cyclohexyl ring;
R⁸ is fluoro;
with the proviso that (3R)-N-[2-cyano-4-fluoro-3-(3-methyl-4-oxo-quinazolin-6-yl)oxyphenyl]-3-fluoro-pyrrolidine-1-sulfonamide is excluded
or a pharmaceutically acceptable salt thereof.

A particular embodiment of the present invention provides a compound according to formula (I) as described herein, wherein R¹ is C₁₋₆-alkyl.

A particular embodiment of the present invention provides a compound according to formula (I) as described herein, wherein R¹ is methyl.

A particular embodiment of the present invention provides a compound according to formula (I) as described herein, wherein R² is selected from
i) H,
ii) chloro, and
iii) fluoro.

A particular embodiment of the present invention provides a compound according to formula (I) as described herein, wherein R² is selected from H and chloro.

A particular embodiment of the present invention provides a compound according to formula (I) as described herein, wherein R³ is NR⁴R⁵.

A particular embodiment of the present invention provides a compound according to formula (I) as described herein, wherein R³ is CHR⁶R⁷.

A particular embodiment of the present invention provides a compound according to formula (I) as described herein, wherein R⁴ is methyl.

A particular embodiment of the present invention provides a compound according to formula (I) as described herein, wherein R⁵ is ethyl.

A particular embodiment of the present invention provides a compound according to formula (I) as described herein, wherein R⁴ and R⁵ together with the nitrogen atom to which they are attached form an heterocycloalkyl optionally substituted with R⁸, wherein the heterocycloalkyl is selected from pyrrolidinyl and piperidinyl.

A particular embodiment of the present invention provides a compound according to formula (I) as described herein, wherein R⁴ and R⁵ together with the nitrogen atom to which they are attached form a pyrrolidinyl ring optionally substituted with R⁸.

A particular embodiment of the present invention provides a compound according to formula (I) as described herein, wherein R⁴ and R⁵ together with the nitrogen atom to which they are attached form an unsubstituted heterocycloalkyl, wherein the heterocycloalkyl is pyrrolidinyl.

A particular embodiment of the present invention provides a compound according to formula (I) as described herein, wherein R⁶ and R⁷ are independently selected from
i) methyl,
ii) ethyl,
iii) propyl,
iv) cyclopropyl, and
v) cyclopropylmethyl.

A particular embodiment of the present invention provides a compound according to formula (I) as described herein, wherein R⁶ is methyl.

A particular embodiment of the present invention provides a compound according to formula (I) as described herein, wherein R⁷ is ethyl.

A particular embodiment of the present invention provides a compound according to formula (I) as described herein, wherein R⁶ and R⁷ together with the nitrogen atom to which they are attached form a cyclopentyl or a cyclohexyl ring optionally substituted with R⁸.

A particular embodiment of the present invention provides a compound according to formula (I) as described herein, wherein R⁶ and R⁷ together with the nitrogen atom to which they are attached form a cyclopentyl or a cyclohexyl ring.

A particular embodiment of the present invention provides a compound according to formula (I) as described herein, wherein R⁸ is fluoro.

A particular embodiment of the present invention provides a compound according to formula (I) as described herein, wherein the compound is selected from
6-[2-cyano-3-[[ethyl(methyl)sulfamoyl]amino]-6-fluoro-phenoxy]-3-methyl-4-oxo-quinazoline;
6-[6-chloro-2-cyano-3-[[ethyl(methyl)sulfamoyl]amino]phenoxy]-3-methyl-4-oxo-quinazoline;
(3R)-N-[4-chloro-2-cyano-3-(3-methyl-4-oxo-quinazolin-6-yl)oxy-phenyl]-3-fluoro-pyrrolidine-1-sulfonamide;
N-[4-chloro-2-cyano-3-(3-methyl-4-oxo-quinazolin-6-yl)oxy-phenyl]pyrrolidine-1-sulfonamide;
6-[2-cyano-3-[[ethyl(methyl)sulfamoyl]amino]phenoxy]-3-methyl-4-oxo-quinazoline;
N-[2-cyano-3-(3-methyl-4-oxo-quinazolin-6-yl)oxy-phenyl]pyrrolidine-1-sulfonamide;
(3R)-N-[2-cyano-3-(3-methyl-4-oxo-quinazolin-6-yl)oxy-phenyl]-3-fluoro-pyrrolidine-1-sulfonamide;
N-[2-cyano-3-(3-methyl-4-oxo-quinazolin-6-yl)oxy-phenyl]cyclopentanesulfonamide;
N-[2-cyano-3-(3-methyl-4-oxo-quinazolin-6-yl)oxy-phenyl]cyclohexanesulfonamide;
N-[2-cyano-3-(3-methyl-4-oxo-quinazolin-6-yl)oxy-phenyl]butane-2-sulfonamide;
6-[2-cyano-3-[[ethyl(methyl)sulfamoyl]amino]anilino]-3-methyl-4-oxo-quinazoline;
(3R)-N-[2-cyano-3-[(3-methyl-4-oxo-quinazolin-6-yl)amino]phenyl]-3-fluoro-pyrrolidine-1-sulfonamide;
6-[6-chloro-2-cyano-3-[[ethyl(methyl) sulfamoyl]amino]anilino]-3-methyl-4-oxo-quinazoline;
N-[4-chloro-2-cyano-3-[(3-methyl-4-oxo-quinazolin-6-yl)amino]phenyl]pyrrolidine-1-sulfonamide;
N-[2-cyano-4-fluoro-3-(3-methyl-4-oxo-quinazolin-6-yl)oxy-phenyl]pyrrolidine-1-sulfonamide;
N-[2-cyano-4-fluoro-3-(3-methyl-4-oxo-quinazolin-6-yl)oxy-phenyl]cyclopentanesulfonamide; and
6-[2-cyano-3-(dimethylsulfamoylamino)-6-fluoro-phenoxy]-3-methyl-4-oxo-quinazoline;
or a pharmaceutically acceptable salt thereof.

A particular embodiment of the present invention provides a compound according to formula (I) as described herein, wherein the compound is selected from
6-[2-cyano-3-[[ethyl(methyl)sulfamoyl]amino]-6-fluoro-phenoxy]-3-methyl-4-oxo-quinazoline;
6-[6-chloro-2-cyano-3-[[ethyl(methyl)sulfamoyl]amino]phenoxy]-3-methyl-4-oxo-quinazoline;
N-[4-chloro-2-cyano-3-(3-methyl-4-oxo-quinazolin-6-yl)oxy-phenyl]pyrrolidine-1-sulfonamide;
6-[2-cyano-3-[[ethyl(methyl)sulfamoyl]amino]phenoxy]-3-methyl-4-oxo-quinazoline;
N-[2-cyano-3-(3-methyl-4-oxo-quinazolin-6-yl)oxy-phenyl]pyrrolidine-1-sulfonamide;
N-[2-cyano-3-(3-methyl-4-oxo-quinazolin-6-yl)oxy-phenyl]cyclopentanesulfonamide;
N-[2-cyano-3-(3-methyl-4-oxo-quinazolin-6-yl)oxy-phenyl]cyclohexanesulfonamide;
N-[2-cyano-3-(3-methyl-4-oxo-quinazolin-6-yl)oxy-phenyl]butane-2-sulfonamide;
6-[2-cyano-3-[[ethyl(methyl)sulfamoyl]amino]anilino]-3-methyl-4-oxo-quinazoline;
6-[6-chloro-2-cyano-3-[[ethyl(methyl)sulfamoyl]amino]anilino]-3-methyl-4-oxo-quinazoline;
N-[4-chloro-2-cyano-3-[(3-methyl-4-oxo-quinazolin-6-yl)amino]phenyl]pyrrolidine-1-sulfonamide;
N-[2-cyano-4-fluoro-3-(3-methyl-4-oxo-quinazolin-6-yl)oxy-phenyl]pyrrolidine-1-sulfonamide;
N-[2-cyano-4-fluoro-3-(3-methyl-4-oxo-quinazolin-6-yl)oxy-phenyl]cyclopentanesulfonamide; and
6-[2-cyano-3-(dimethylsulfamoylamino)-6-fluoro-phenoxy]-3-methyl-4-oxo-quinazoline;
or a pharmaceutically acceptable salt thereof.

A particular embodiment of the present invention provides a compound according to formula (I) as described herein, wherein the compound is selected from
6-[6-chloro-2-cyano-3-[[ethyl(methyl)sulfamoyl]amino]phenoxy]-3-methyl-4-oxo-quinazoline;
N-[4-chloro-2-cyano-3-(3-methyl-4-oxo-quinazolin-6-yl)oxy-phenyl]pyrrolidine-1-sulfonamide;
6-[2-cyano-3-[[ethyl(methyl)sulfamoyl]amino]phenoxy]-3-methyl-4-oxo-quinazoline;
N-[2-cyano-3-(3-methyl-4-oxo-quinazolin-6-yl)oxy-phenyl]pyrrolidine-1-sulfonamide;
N-[2-cyano-3-(3-methyl-4-oxo-quinazolin-6-yl)oxy-phenyl]cyclopentanesulfonamide;
N-[2-cyano-3-(3-methyl-4-oxo-quinazolin-6-yl)oxy-phenyl]cyclohexanesulfonamide;
N-[2-cyano-3-(3-methyl-4-oxo-quinazolin-6-yl)oxy-phenyl]butane-2-sulfonamide;
6-[2-cyano-3-[[ethyl(methyl)sulfamoyl]amino]anilino]-3-methyl-4-oxo-quinazoline;
6-[6-chloro-2-cyano-3-[[ethyl(methyl)sulfamoyl]amino]anilino]-3-methyl-4-oxo-quinazoline; and
N-[4-chloro-2-cyano-3-[(3-methyl-4-oxo-quinazolin-6-yl)amino]phenyl]pyrrolidine-1-sulfonamide;
or a pharmaceutically acceptable salt thereof.

The term "C₁₋₆-alkyl", alone or in combination, denotes a monovalent linear or branched saturated hydrocarbon group of 1 to 6 carbon atoms. Examples of C₁₋₆-alkyl include methyl, ethyl, propyl, isopropyl, n-butyl, iso-butyl, sec-butyl, tert-butyl and pentyl. Particular C₁₋₆-alkyl groups are methyl, ethyl, propyl and n-butyl. More particular C₁₋₆-alkyl groups are methyl, ethyl and propyl.

The term "C₃₋₈-cycloalkyl", alone or in combination, denotes a monovalent saturated monocyclic or bicyclic hydrocarbon group of 3 to 8 ring carbon atoms. Bicyclic means a ring system consisting of two saturated carbocycles having on or two carbon atoms in common. Examples of monocyclic C₃₋₈-cycloalkyl are cyclopropyl, cyclobutanyl, cyclopentyl, cyclohexyl or cycloheptyl. Particular monocyclic cycloalkyl groups are cyclopropyl, cyclopentyl and cyclohexyl.

The term "C₃₋₈-cycloalkyl-C₁₋₆-alkyl", alone or in combination, denotes an -C₁₋₆-alkyl group wherein one of the hydrogen atoms of the C₁₋₆-alkyl group has been replaced by an C₃₋₈-cycloalkyl group. Examples of C₃₋₈-cycloalkyl-C₁₋₆-alkyl include cyclopropylmethyl, cyclopropylethyl, cyclopropylbutyl, cyclobutylpropyl, 2-cyclopropylbutyl, cyclopentylbutyl, cyclohexylmethyl, cyclohexylethyl, bicyclo[4.1.0]heptanylmethyl, bicyclo[4.1.0]heptanylethyl, bicyclo[2.2.2]octanylmethyl and bicyclo[2.2.2]octanylethyl. A particular example of C₃₋₈-cycloalkyl-C₁₋₆-alkyl is cyclopropylmethyl.

The term "halogen" and "halo", alone or in combination, are used interchangeably herein and denote fluoro, chloro, bromo or iodo. Particular examples of halogen are chloro and fluoro. A particular halogen is fluoro.

The term "heterocycloalkyl" denotes a monovalent saturated or partly unsaturated mono- or bicyclic ring system of 4 to 9 ring atoms, comprising 1, 2, or 3 ring heteroatoms selected from N, O and S, the remaining ring atoms being carbon. Bicyclic means consisting of two cycles having one or two ring atoms in common. Examples for monocyclic saturated heterocycloalkyl are 4,5-dihydro-oxazolyl, oxetanyl, azetidinyl, pyrrolidinyl, 2-oxo-pyrrolidin-3-yl, tetrahydrofuranyl, tetrahydrothiophenyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are oxabicyclo[2.2.1]heptanyl, oxaspiro[3.3]heptanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl. Particular heterocycloalkyl are pyrrolidinyl and piperidinyl.

Processes for the manufacture of the compound of formula (I) as described herein are also an object of the invention.

The preparation of the compound of formula (I) of the present invention may be carried out in sequential or convergent synthetic routes. Syntheses of the invention is shown in the following general scheme. The skills required for carrying out the reactions and purifications of the resulting products are known to those skilled in the art. The substituents and indices used in the following description of the processes have the significance given herein unless indicated to the contrary.

In more detail, the compound of formula (I) can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

### General synthesis of compounds

Compounds of formula (I) can be prepared by the reaction of aryl fluorides of formula A with sulfonamides or sulfamides **B** in the presence of a base such as Cs₂CO₃ or NaH in a solvent such as DMF or NMP (Scheme 1).

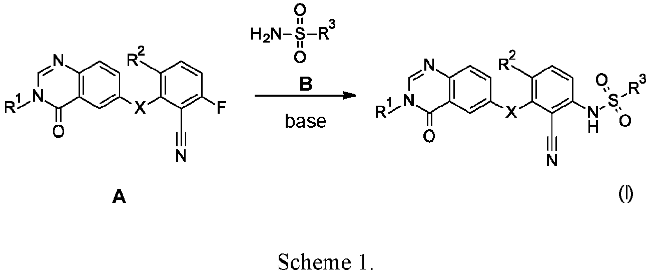

Compounds of formula (I) can additionally be prepared by the reaction of anilines C with sulfonyl chlorides or sulfamoyl chlorides **D** in the presence of a base such as pyridine in a solvent such as DCM (Scheme 2).

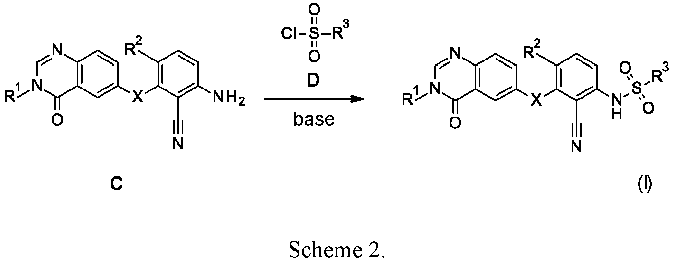

Compounds of formula (I) when X = NH can additionally be prepared by the reaction of bromides **E** with anilines **F** in the presence of a base such as Cs₂CO₃, a palladium catalyst such as tris(dibenzylideneacetone)dipalladium (0) and a ligand such as BippyPhos in a solvent such as dioxane (Scheme 3).

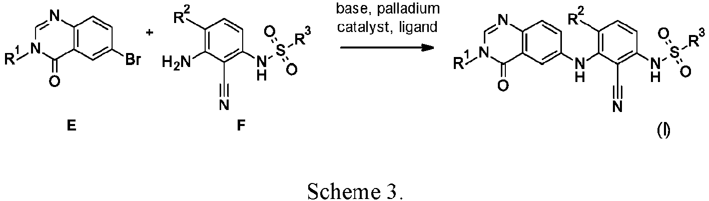

Intermediates **A** where X = O can be prepared by condensing 2-amino-5-hydroxybenzoic acid and N-alkylformamides **G,** for example by heating in the absence of solvent, to afford 6-hydroxy-quinazolin-4-ones **H,** which can react with fluorobenzonitriles I in the presence of a base such as NaH or Cs₂CO₃ in a solvent such as DMF or NMP (Scheme 4).

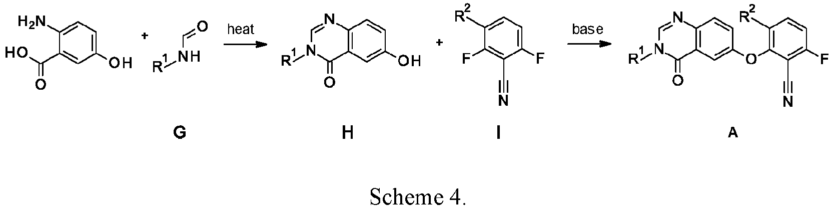

Intermediates **C** can be prepared by the reaction of intermediates **A** with aqueous ammonia in a solvent such as 2-propanol (Scheme 5).

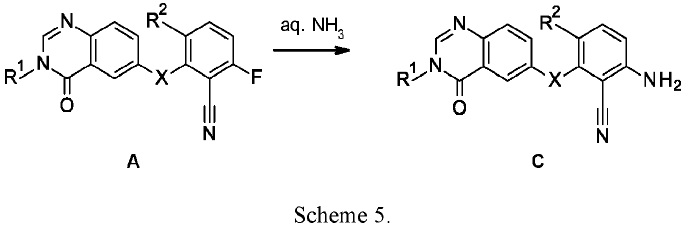

Intermediates **C** where X = NH can be prepared by the reduction of 2,6-dinitrobenzonitriles **J** with a reducing agent such as iron in aqueous HCl in a solvent such as a mixture of methanol and dioxane, to give 2,6-diaminobenzonitriles **K.** Subsequent reaction with 6-bromoquinazolin-4-ones **E** in the presence of a catalyst such as tris(dibenzylideneacetone)dipalladium (0), a ligand such as BippyPhos and a base such as Cs₂CO₃, in a solvent such as dioxane gives intermediates **C** (Scheme 6).

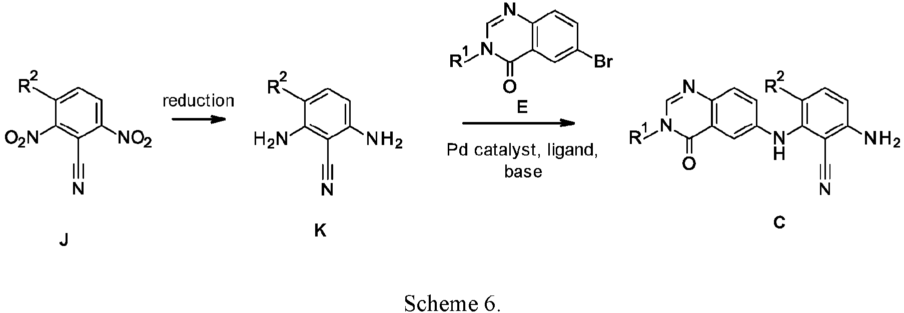

Intermediates **C** where X = NH can also be prepared as shown in Scheme 7. 2-Amino-6-nitrobenzoic acid and an *N*-alkylformamide **G** are condensed to form 6-nitroquinazolin-4-ones **L,** for example by heating in the absence of a solvent, which is then reduced to the 6-aminoquinazolin-4-one **M,** for example by using hydrogen and a catalyst (such as palladium on carbon) in a solvent such as a mixture of methanol and acetic acid. Reaction with fluorobenzonitriles **I** in the presence of a base such as potassium tert-butoxide in a solvent such as DMSO affords intermediates **N.** Reaction with an azide salt such as NaN₃ in a solvent such as DMF affords azides **O,** which can be reduced to the amine **C** (for example, by using the procedure described in Pei and Wickham, Tetrahedron Lett. (1993), 34, 7509-7512).

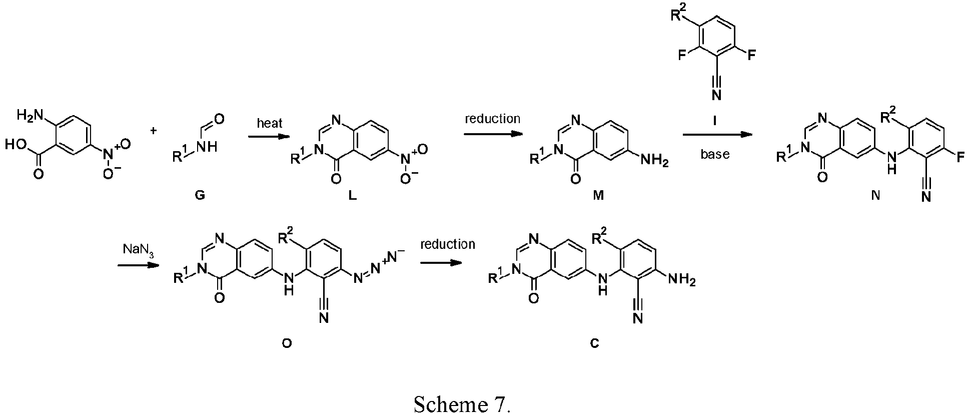

Intermediates **F** can be prepared by treatment of 2,6-dinitrobenzonitrile **P** with sulfonamides or sulfamides **B** in the presence of a base such as Cs₂CO₃ in a solvent such as DMF. The resultant nitro compounds **Q** can be reduced, for example by hydrogenation with a catalyst such as Pd(OH)₂ in a solvent such as a mixture of methanol and THF (Scheme 8).

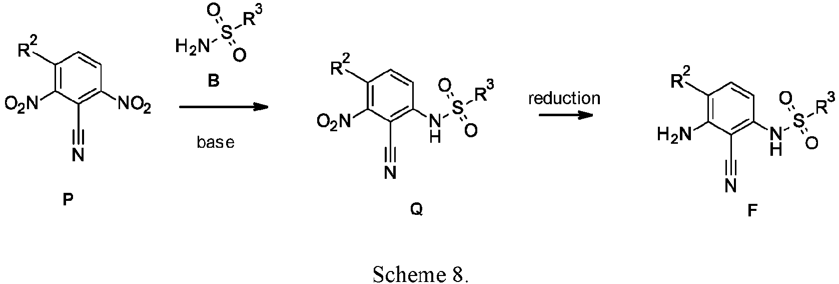

Intermediates **B,** where R³ is of the type NR⁴R⁵ (i.e. sulfamides), where not commercially available, can be prepared from the reaction of sulfuric diamide with an amine **R** in dioxane, in the presence of absence of a base such as triethylamine (Scheme 9).

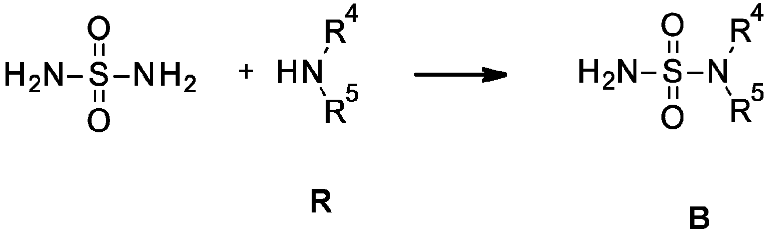

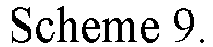

Intermediates **B,** where R³ is of the type CHR⁶R⁷ (i.e. sulfonamides), where not commercially available, can be prepared from the corresponding sulfonyl chlorides S by reaction with aqueous ammonia (Scheme 10).

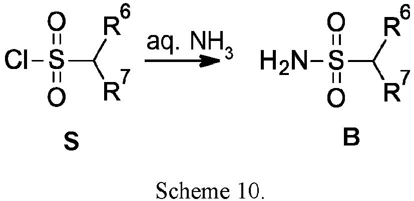

Intermediates **D,** where R³ is of the type NR⁴R⁵ (i.e. sulfamoyl chlorides), where not commercially available, can be prepared from a secondary amine T and sulfuoryl dichloride in the presence of a base such as DIPEA in a solvent such as DCM (Scheme 11).

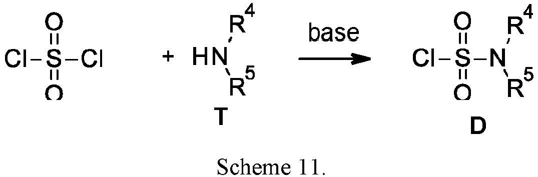

It will be appreciated that the compound of formula (I) in this invention may be derivatised at functional groups to provide derivatives which are capable of conversion back to the parent compound *in vivo.*

The invention also relates in particular to:
A compound of formula (I) as described herein, or a pharmaceutically acceptable salt thereof, for use as therapeutically active substance;
A pharmaceutical composition comprising a compound of formula (I) as described herein, or a pharmaceutically acceptable salt thereof, and a therapeutically inert carrier;
A compound of formula (I) as described herein, or a pharmaceutically acceptable salt thereof, for use in the treatment or prophylaxis of cancer;
A compound of formula (I) as described herein, or a pharmaceutically acceptable salt thereof, for use in the treatment or prophylaxis of thyroid cancer, colorectal cancer, brain cancer, melanoma or non-small cell lung cancer (NSCLC);

A certain embodiment of the invention relates to the compound of formula (I) as described herein, or a pharmaceutically acceptable salt thereof, for the use in the therapeutic and/or prophylactic treatment of cancer, in particular BRAF mutant driven cancers, more particularly thyroid cancer, colorectal cancer, brain cancer, melanoma or NSCLC.

A certain embodiment of the invention relates to a pharmaceutical composition comprising the compound of formula (I) as described herein, or a pharmaceutically acceptable salt thereof, and a pharmaceutically acceptable excipient.

A certain embodiment of the invention relates to the compound of formula (I) as described herein, or a pharmaceutically acceptable salt thereof, for the use as a medicament in therapeutic and/or prophylactic treatment of a patient with BRAF mutant driven cancer, in particular more particularly thyroid cancer, colorectal cancer, brain cancer, melanoma or NSCLC comprising determining the BRAF mutation status in said patient and then administering the compound of formula (I) as described herein, or a pharmaceutically acceptable salt thereof, to said patient.

Furthermore, the invention includes all substituents in their corresponding deuterated form, wherever applicable, of the compound of formula (I).

A certain embodiment of the invention relates to the compound of formula (I) as described herein, or a pharmaceutically acceptable salt thereof, wherein at least one substituent comprises at least one radioisotope. Particular examples of radioisotopes are ²H, ³H, ¹³C, ¹⁴C and ¹⁸F.

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

The compound of formula (I) may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

### Assay procedures

### Materials

DMEM no-phenol red medium supplemented with L-glutamine was purchased from (Thermo Fisher Scientific). Fetal bovine serum (FBS) was purchased from VWR. Advanced ERK phospho-T202 /Y204 kit - 10,000 tests was purchased from Cisbio cat# 64AERPEH. A375 and HCT116 cells were originally obtained from ATCC and banked by the Roche repository. 384-well microplates were purchased from Greiner Bio-One, 384-well, (With Lid, HiBase, Low volume cat 784-080).

### HTRF assay for P-ERK determination in A375 or HCT116 cells

A375 is a cellular cancer model expressing V600E mutated BRAF and HCT116 a cellular cancer model expressing WT BRAF. First generation BRAF inhibitors such as e.g. dabrafenib induce a paradox effect on tumour cells in that they inhibit the growth of V600E mutated BRAF cells (such as e.g. A375), while they activate growth in WT BRAF cells (such as e.g. HCT 116). ERK 1,2 phosphorylation (terminal member of the phosphorylation cascade of the MAPK pathway) is hereafter reported as main readout for the activation status of the MAPK pathway. Prior to the assay, A375 and HCT116 cell lines are maintained in DMEM no-phenol red medium supplemented with 10% fetal bovine serum (FBS). Following compound treatment, P-ERK levels are determined by measuring FRET fluorescence signal induced by selective binding of 2 antibodies provided in the mentioned kit (Cisbio cat# 64AERPEH) on ERK protein when phosphorylated at Thr202/Tyr204. Briefly, 8000 cells/well in 12 µl media/well are plated in the 384-well plate and left overnight in the incubator (at 37 °C with 5% CO2-humidified atmosphere), the following day the plate is treated in duplicate with test compounds, dabrafenib and PLX8394 (the latter two as controls) at the following final drug concentrations: 10µM-3µM-1µM-0.3µM-0.1µM-0.03µM-0,01µM-0.003µM-0.001µM, all wells are subjected to DMSO normalization and drug incubation occurs for 1 hour. Then, 4µl of a 4X lysis buffer supplied with the kit are added to the wells, the plate is then centrifuged for 30 second (300 rcf) and incubated on a plate shaker for 1h at RT.

At the end of the incubation 4µL/well of advanced P-ERK antibody solution (prepared according to manufacturer's instruction) followed by 4µL/well of criptate P-ERK antibody solution (prepared according to manufacturer's instruction) (Cisbio cat# 64AERPEH) are added to test wells.

In order to allow proper data normalization control wells non drug treated reported in the following table are always included in each plate (according to manufacturer's instruction):
p-ERK HTRF well compositions (µl):

| neg ctrl | pos ctrl | neut ctrl | cpd | blank | |
|---|---|---|---|---|---|
| - | - | 12 | 12 | 12 | Cells |
| 12 | - | - | - | - | Media |
| - | - | - | <0.05 | - | Cpd |
| - | 16 | - | - | - | control lysate (ready-to-use) |
| 4 | - | 4 | 4 | 4 | 4x lysis buffer |
| 4 | 4 | 4 | 4 | | Advanced p-ERK antibody solution |
| - | - | - | - | 4 | Advanced p-ERK1/2 Cryptate antibody solut. |
| 20 | 20 | 20 | 20 | 20 | Total volume in Well |

The plate is then centrifuged at 300 rcf for 30 second, sealed to prevent evaporation and incubated overnight in the dark at room temperature.

The plate is then analyzed and fluorescence emission value collected through a Pherastast FSX (BMG Labtech) apparatus at 665 and 620 nM.

The obtained fluorescence values are processed according to the formula Ratio=Signal(620nm)/Signal(625nm)*10000 then the average of the ratio on the blank is subtracted to all values.

Data are normalized in the case of A375 cells (BRAF inhibition) considering the average of the ratio (blank subtracted) derived by DMSO only treated cells as 100% and by considering the average of the ratio (blank subtracted) derived by 10uM dabrafenib treated cells as 0%. Mean of the normalized points are fitted with sigmoidal curve and IC50 determined.

Data are normalized in the case of HCT116 cells (BRAF activation) considering the average of the ratio (blank subtracted) derived by DMSO only treated cells as 0% and by considering the average of the ratio (blank subtracted) derived by dabrafenib treated cells at the concentration which provides the highest signal as 100%. Individual points are fitted with either sigmoidal or bell shape curves, and the percentage of activation compared to maximum dabrafenib-mediated activation is determined. The EC50 is the concentration at which activation equal to 50% of the maximum achieved by dabrafenib is obtained.

In case the activation does not reach 50% of the maximum achieved by dabrafenib, then the EC50 calculation is not applicable.

The Percentage of Maximum paradox inducing effect from dabrafenib is determined by evaluating the percentage at which the test compound induce its maximum P-ERK signal as percentage of the highest signal produced by dabrafenib within the dose range tested.

**Table 1: Examples 1 to 17 have high affinity for RAF kinases.**

| **Example** | **Kd (µM)** | | |
|---|---|---|---|
| | **BRAF (V600E)** | **BRAF** | **CRAF** |
| **1** | 0.0034 | 0.0016 | 0.0063 |
| **2** | 0.0368 | 0.0304 | 0.0054 |
| **3** | 0.0063 | 0.01 | 0.0094 |
| **4** | 0.0046 | 0.0021 | 0.0016 |
| **5** | 0.0323 | 0.0106 | 0.0072 |
| **6** | 0.0026 | 0.0016 | 0.0011 |
| **7** | 0.0045 | 0.0089 | 0.0089 |
| **8** | 0.0070 | 0.0033 | 0.0018 |
| **9** | 0.0040 | 0.0024 | 0.0008 |
| **10** | 0.0144 | 0.0039 | 0.0068 |
| **11** | 0.0265 | 0.0116 | 0.0523 |
| **12** | 0.0075 | 0.0020 | 0.0098 |
| **13** | 0.0100 | 0.0043 | 0.0068 |
| **14** | 0.0007 | 0.0014 | 0.0015 |
| **15** | 0.0009 | 0.0004 | 0.0008 |
| **16** | 0.0019 | 0.0010 | 0.0007 |
| **17** | 0.0043 | 0.0017 | 0.003 |
| **AR-25** | 0.0001 | 0.0002 | 0.0003 |
| **AR-30** | 0.1740 | 0.5040 | 0.8220 |
| **AR-31** | 0.0459 | 0.1190 | 0.1903 |

**Table 2: The results of Table 2 demonstrate that the compounds of the invention break the paradoxical RAF activation in HCT-116 cancer cells expressing WT BRAF. When compared with dabrafenib or with AR-25, the maximum paradox inducing effect is substantially reduced by more than 25% for all examples.**

| **Example** | **pERK IC₅₀ (nM)** | **p-ERK EC50 (nM)** | **Percentage of maximum paradox inducing effect from dabrafenib** |
|---|---|---|---|
| | | **conc. at which the compound induces p-ERK activation of 50% of that induced by dabrafenib (Positive control paradox inducer)** | |
| | **A375** | **HCT-116** | |
| **1** | 5.5 | >1000 | 56 |
| **2** | 28.6 | >1000 | 69 |
| **3** | 14.8 | >1000 | 61 |
| **4** | 34.3 | 714 | 65 |
| **5** | 52.5 | >1000 | 61 |
| **6** | 17.1 | 648 | 59 |
| **7** | 26.2 | not applicable | 32 |
| **8** | 5.3 | 412 | 63 |
| **9** | 13.9 | not applicable | 44 |
| **10** | 8.8 | not applicable | 21 |
| **11** | 19.7 | >1000 | 61 |
| **12** | 9.2 | not applicable | 49 |
| **13** | 14.8 | >1000 | 67 |
| **14** | 13.7 | 204 | 74 |
| **15** | 1.6 | 172 | 63 |
| **16** | 2.3 | 152 | 70 |
| **17** | 2.6 | 970 | 71 |
| **AR-25** | 1.1 | 9.6 | 103 |
| **AR-30** | 406 | >1000 | 59 |
| **AR-31** | 311 | >1000 | 51 |

WO2012/118492 discloses references compounds AR-25 as example 25, AR-30 as example 30 and AR-31 as example 31.

The compound of formula (I) or a pharmaceutically acceptable salt thereof can be used as a medicament (e.g. in the form of a pharmaceutical preparation). The pharmaceutical preparation can be administered internally, such as orally (e.g. in the form of tablets, coated tablets, dragées, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compound of formula (I) or a pharmaceutically acceptable salt thereof can be processed with pharmaceutically inert, inorganic or organic adjuvants for the production of tablets, coated tablets, dragées, hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

### Pharmaceutical Compositions

The compound of formula (I) or a pharmaceutically acceptable salt thereof can be used as therapeutically active substance, e.g. in the form of a pharmaceutical preparation. The pharmaceutical preparation can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compound of formula (I) and pharmaceutically acceptable salts thereof can be processed with a pharmaceutically inert, inorganic or organic carrier for the production of a pharmaceutical preparation. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparation can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing the compound of formula (I) or a pharmaceutically acceptable salt thereof and a therapeutically inert carrier are also provided by the present invention, as is a process for their production, which comprises bringing one or more compounds of formula (I) and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration the dosage for adults can vary from about 0.01 mg to about 1000 mg per day of a compound of general formula (I) or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, particularly 1-100 mg, of a compound of formula (I). Examples of compositions according to the invention are:

### Example A

Tablets of the following composition are manufactured in the usual manner:

**Table 1: possible tablet composition**

| ingredient | mg/tablet | | | |
|---|---|---|---|---|
| | 5 | 25 | 100 | 500 |
| Compound of formula (I) | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 2: possible capsule ingredient composition**

| ingredient | mg/capsule | | | |
|---|---|---|---|---|
| | 5 | 25 | 100 | 500 |
| Compound of formula (I) | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula (I), lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 3: possible soft gelatin capsule ingredient composition**

| ingredient | mg/capsule |
|---|---|
| Compound of formula (I) | 5 |
| Yellow wax | 8 |
| Hydrogenated Soya bean oil | 8 |
| Partially hydrogenated plant oils | 34 |
| Soya bean oil | 110 |
| Total | 165 |

**Table 4: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula (I) is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 5: possible suppository composition**

| ingredient | mg/supp. |
|---|---|
| Compound of formula (I) | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula (I) is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 6: possible injection solution composition**

| ingredient | mg/injection solution. |
|---|---|
| Compound of formula (I) | 3 |
| Polyethylene Glycol 400 | 150 |
| acetic acid | q.s. ad pH 5.0 |
| water for injection solutions | ad 1.0 ml |

### Manufacturing Procedure

The compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 7: possible sachet composition**

| ingredient | mg/sachet |
|---|---|
| Compound of formula (I) | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula (I) is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Examples

The following examples are provided for illustration of the invention. They should not be considered as limiting the scope of the invention, but merely as being representative thereof.

### Abbreviations

AcOH = acetic acid; DCM = dichloromethane; DIPEA = diisopropylethylamine; DMAP = dimethylaminopyridine; DMF = dimethylformamide; DMSO = dimethyl sulfoxide; ESI = electrospray ionization; EtOAc = ethyl acetate; EtOH = ethanol; GTP = guanosine triphosphate; HATU = hexafluorophosphate azabenzotriazole tetramethyl uronium; HPLC = high performance liquid chromatography; MeOH = methanol; MS = mass spectrometry; NMP = N-methyl-2-pyrrolidone; NMR = nuclear magnetic resonance; rt = room temperature; THF = tetrahydrofuran; TRIS = tris(hydroxymethyl)aminomethane.

### Intermediate I1: 6-hydroxy-3-methyl-quinazolin-4-one

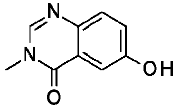

2-Amino-5-hydroxybenzoic acid (10 g, 65.3 mmol, Eq: 1.0) and N-methylformamide (30 g, 29.9 mL, 503 mmol, Eq: 7.7) were heated at 145 °C for 21 h 45 min, then cooled to rt. The reaction mixture was diluted with 50 mL H₂O and stirred at rt for 20 min. The resulting precipitate was collected by filtration. The light brown solid was washed 3 × with 20 mL water. The solid was taken up in toluene and evaporated to dryness (3 ×). The solid was dried in vacuo at 40 °C overnight under high vacuum to give the title compound as a light brown solid (10.3 g, 89% yield). MS (ESI) *m*/*z:* 177.1 [M+H]⁺.

### Intermediate I2: 3,6-difluoro-2-(3-methyl-4-oxo-quinazolin-6-yl)oxy-benzonitrile

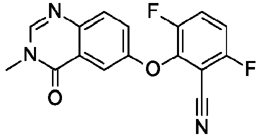

Cesium carbonate (3.22 g, 9.79 mmol, Eq: 1.15) was added at rt to a solution of **I1** (1500 mg, 8.51 mmol, Eq: 1) in *N*,*N*-dimethylformamide (35 mL). The mixture was stirred for 30 min at rt then 2,3,6-trifluorobenzonitrile (1.47 g, 1.08 ml, 9.37 mmol, Eq: 1.1) was added. After 1 h, the reaction was cooled on ice and diluted with water (120 mL). The resultant solid was collected by filtration, washed with iced water (100 mL) and heptane (100 mL) and suction-dried. The solid was taken up in toluene and evaporated to dryness (3 ×) then dried overnight in vacuo to give the title compound as a light brown solid (2.58 g, 97% yield). MS (ESI) *m*/*z:* 314.1 [M+H]⁺.

### Intermediate I3: 2-fluoro-6-(3-methyl-4-oxo-quinazolin-6-yl)oxy-benzonitrile

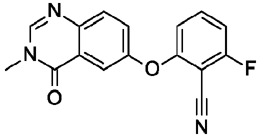

NaH (60% in mineral oil, 285 mg, 6.53 mmol, Eq: 1.15) was added at 0 °C to a solution of **I1** (6-hydroxy-3-methylquinazolin-4-one) (1.00 g, 5.68 mmol, Eq: 1.0) in DMF (15 mL). The cooling bath was removed, and the reaction was stirred at rt for 15 min. The reaction was again cooled to 0 °C, and 2,6-difluorobenzonitrile (790 mg, 5.68 mmol, Eq: 1.0) in DMF (2.5 mL) was added. The reaction was warmed to rt and stirred under argon for 2 h. After 2 h, the reaction was cooled on ice, and water (100 mL) was added. The resultant precipitate was collected by filtration, and washed with water and heptanes to give the title compound as a beige solid (1.62 g, 93% purity, 90% yield). MS (ESI) *m*/*z:* 296.1 [M+H]⁺.

### Intermediate I4: 6-amino-3-chloro-2-(3-methyl-4-oxo-quinazolin-6-yl)oxy-benzonitrile

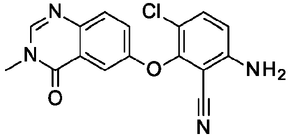

### Step 1: 3-chloro-6-fluoro-2-(3-methyl-4-oxo-quinazolin-6-yl)oxy-benzonitrile

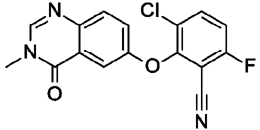

3-Chloro-2,6-difluorobenzonitrile (200 mg, 1.15 mmol, Eq: 1.0), **I1** (6-hydroxy-3-methylquinazolin-4-one) (203 mg, 1.15 mmol, Eq: 1.0) and K₂CO₃ (319 mg, 2.3 mmol, Eq: 2.0) in NMP (1.15 mL) were heated at 100 °C for 14 h, the cooled to rt. The reaction was diluted with water (30 mL) and extracted with EtOAc (2 × 30 mL). The combined organic layers were washed with brine (3 × 40 mL), dried (Na₂SO₄), filtered and concentrated in vacuo. Purification by flash chromatography (20 g silica, 50-100% EtOAc in heptane) gave the title compound as a colourless solid (231 mg, 100% purity, 61% yield). MS (ESI) m/z: 330.1 [M+H]⁺.

### Step 2: 6-amino-3-chloro-2-(3-methyl-4-oxo-quinazolin-6-yl)oxy-benzonitrile

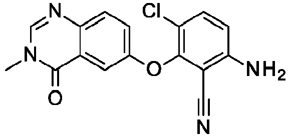

3-Chloro-6-fluoro-2-(3-methyl-4-oxo-quinazolin-6-yl)oxy-benzonitrile (550 mg, 1.67 mmol, Eq: 1.0), ammonium hydroxide (25% in water, 2.7 g, 3 mL, 77 mmol, Eq: 46.2) and 2-propanol (3 mL) were heated in a sealed vial with microwave irradiation for 25 min at 160 °C. The reaction was cooled to rt, and the resultant precipitate was filtered, washed with water, isopropanol and diethyl ether to give the title compound as a colourless solid (426 mg, 96% purity, 78% yield). MS (ESI) *m*/*z:* 327.1 [M+H]⁺.

### Intermediate I5: 1-amino-2-cyano-3-[[ethyl(methyl)sulfamoyl]amino]benzene

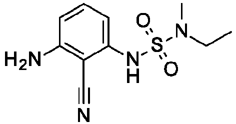

### Step 1: 2-cyano-1-[[ethyl(methyl)sulfamoyl]amino]-3-nitro-benzene

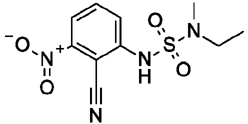

2,6-Dinitrobenzonitrile (1.46 g, 7.56 mmol, Eq: 1.1) was dissolved in DMF (15 mL). Cs₂CO₃ (2.46 g, 7.56 mmol, Eq: 1.1) and [methyl(sulfamoyl)amino]ethane (1 g, 6.87 mmol, Eq: 1.0) were added. The reaction mixture was stirred for 2 h at 65 °C, then concentrated in vacuo. The residue was taken up in 2-methyl-THF and washed with water-brine solution, and the aqueous layer was extracted 2 × with 2-methyl-THF. The organic layers were combined, dried with Na₂SO₄, filtered and concentrated in vacuo. The residue was diluted with DCM, evaporated with silica gel to dryness and transferred to a column. Purification by flash chromatography (40 g silica, 0-100% EtOAc in DCM) gave the title compound as a light red viscous oil (720 mg, 77% purity) which was used without further purification. MS (ESI) *m*/*z:* 285.1 [M+H]⁺.

### Step 2: 1-amino-2-cyano-3-[[ethyl(methyl)sulfamoyl]amino]benzene

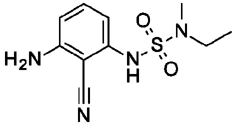

1-Amino-2-cyano-3-[[ethyl(methyl)sulfamoyl]amino]benzene (703 mg, 1.9 mmol, Eq: 1.0) was dissolved in MeOH (17 mL) and THF (7 mL), then Pd(OH)₂ (Pearlman's catalyst, 26.7 mg, 190 µmol, Eq: 0.1) was added and the reaction mixture was stirred under a balloon of hydrogen at rt. After 1 h, the reaction mixture was filtered over a Whatman Spartan 30/0.45RC filter, and the filtrate was evaporated. The residue was diluted with EtOAc and transferred to a column. Purification by flash chromatography (80 g silica, 0-68% EtOAc in heptane) gave the title compound as a viscous orange oil (457 mg, 100% purity, 27% yield over two steps). MS (ESI) *m*/*z:* 255.1 [M+H]⁺.

### Intermediate I6: (3R)-N-(3-amino-2-cyano-phenyl)-3-fluoro-pyrrolidine-1-sulfonamide

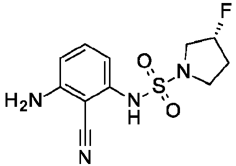

### Step 1: (3R)-N-(2-cyano-3-nitro-phenyl)-3-fluoro-pyrrolidine-1-sulfonamide

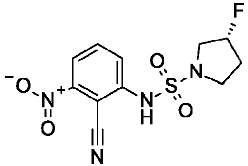

2,6-Dinitrobenzonitrile (900 mg, 4.66 mmol, Eq: 1.0) was dissolved in DMF (10 mL). Cs₂CO₃ (2.28 g, 6.99 mmol, Eq: 1.5) and **I9** (1.18 g, 6.99 mmol, Eq: 1.5) were added. The reaction mixture was stirred for 1 h at 60 °C, then concentrated in vacuo. The residue was taken up in 2-methyl-THF and washed with aq. NH₄Cl solution, and the aqueous layer was extracted 1 × with 2-methyl-THF. The organic layers were combined, dried with Na₂SO₄, filtrated and concentrated in vacuo. The residue was diluted with DCM, evaporated with silica gel to dryness and transferred to a column. Purification by flash chromatography (40 g silica, 0-100% EtOAc in DCM) gave the title compound as a light red viscous oil (795 mg). MS (ESI) *m*/*z:* 315.1 [M+H]⁺.

### Step 2: (3R)-N-(3-amino-2-cyano-phenyl)-3-fluoro-pyrrolidine-1-sulfonamide

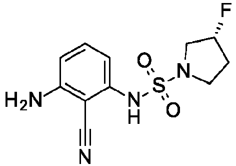

(3*R*)-*N*-(2-Cyano-3-nitro-phenyl)-3-fluoro-pyrrolidine-1-sulfonamide (751 mg, 2.39 mmol, Eq: 1.0) was dissolved in MeOH (13 mL) and THF (6 mL), then Pd(OH)₂ (Pearlman's catalyst , 33.6 mg, 239 µmol, Eq: 0.1) was added and the reaction mixture was stirred under a balloon of hydrogen at rt. After 1 h, the reaction mixture was filtered over a Whatman Spartan 30/0.45RC filter, and the filtrate was evaporated. The residue was diluted with EtOAc and transferred to a column. Purification by flash chromatography (80 g silica, 0-79% EtOAc in heptane) gave the title compound as a viscous orange oil (560 mg, 100% purity, 42% yield over two steps). MS (ESI) *m*/*z:* 285.1 [M+H]⁺.

### Intermediate I7: 2-amino-6-[(3-methyl-4-oxo-quinazolin-6-yl)amino]benzonitrile

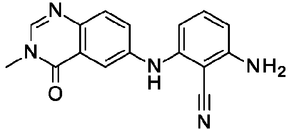

### Step 1: 2,6-diaminobenzonitrile

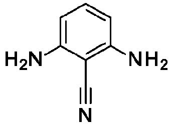

2,6-Dinitrobenzonitrile (3 g, 15.5 mmol, Eq: 1.0) was dissolved in a mixture of methanol (60 mL) and dioxane (35 mL). The reaction was heated to 75 °C, then HCl (37% aq, 11 g, 9.29 mL, 111 mmol, Eq: 7.16) was added dropwise, then iron (2.78 g, 49.7 mmol, Eq: 3.2) was added in 4 portions over 8 min. The reaction mixture was stirred for 1 h at 64 °C, then concentrated in vacuo. The residue was taken up in 2-methyl-THF and ice and washed with sat. aq. NaHCO₃. Both layers were filtered, and the aqueous layer was back-extracted with 2-methyl-THF. The organic layers were combined, washed with brine, dried over Na₂SO₄ and concentrated in vacuo. The residue was diluted with DCM, evaporated with silica gel to dryness and transferred to a column. Purification by flash chromatography (80 g silica, DCM) gave the title compound (268 mg, 13% yield) along with 2-amino-6-nitro-benzonitrile (465 mg, 18% yield). 2,6-diaminobenzonitrile: ¹H NMR (300 MHz, DMSO-*d₆*) δ ppm 5.55 (s, 4 H) 5.89 (d, *J*=8.1 Hz, 2 H) 6.89 (t, *J*=8.1 Hz, 1 H). 2-amino-6-nitro-benzonitrile: ¹H NMR (300 MHz, DMSO-*d₆*) δ ppm 6.74 (br s, 2 H) 7.20 (dd, *J*=8.3, 1.0 Hz, 1 H) 7.40 - 7.55 (m, 2 H).

### Step 2: 2-amino-6-[(3-methyl-4-oxo-quinazolin-6-yl)amino]benzonitrile

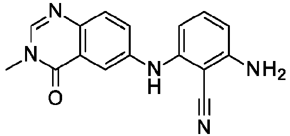

6-Bromo-3-methylquinazolin-4(3H)-one (200 mg, 820 µmol, Eq: 1.0) and 2,6-diaminobenzonitrile (109 mg, 820 µmol, Eq: 1.0) were dissolved in dioxane (10 mL) then Cs₂CO₃ (809 mg, 2.46 mmol, Eq: 3.0) was added. The reaction mixture was flushed with argon, then BippyPhos (25.7 mg, 49.2 µmol, Eq: 0.06) and tris(dibenzylideneacetone)dipalladium (0) chloroform adduct (26 mg, 24.6 µmol, Eq: 0.03) were added. The reaction was flushed with argon again, and the vial was closed. The reaction mixture was heated to 110 °C and stirred for 9.5 h. The reaction mixture was taken up in 15 mL 2-methyl-THF and ice and washed with 4 mL 1% aq. citric acid. The aqueous layer was back-extracted with 1 × 15 mL 2-methyl-THF. The organic layers were combined, washed with brine, dried over Na₂SO₄, filtered and concentrated in vacuo. The residue was diluted with EtOAc and transferred to a column. Purification by flash chromatography (40 g silica, 0-100% EtOAc in heptane) gave the title compound as a light yellow solid (21 mg, 97% purity, 8.6% yield). MS (ESI) *m*/*z:* 292.1 [M+H]⁺.

### Intermediate I8: 6-amino-3-chloro-2-[(3-methyl-4-oxo-quinazolin-6-yl)amino]benzonitrile

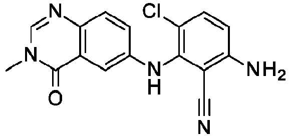

### Step 1: 6-nitro-3-methyl-quinazolin-4-one

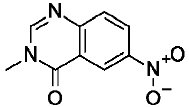

2-Amino-5-nitrobenzoic acid (5 g, 27.5 mmol, Eq: 1.0) and N-methylformamide (15.2 g, 15 mL, 257 mmol, Eq: 9.35) were heated for 4 h at 180 °C in a sealed tube. The reaction was then cooled to rt and poured into ice-cold water (150 mL). The resulting precipitate was collected by filtration and washed with further ice-cold water. The solid was concentrated twice to dryness from toluene, then dried further under high vacuum to give the title compound as a yellow-brown solid (3.17 g, 56% yield). MS (ESI) m/z: 206.1 [M+H]⁺.

### Step 2: 6-amino-3-methyl-quinazolin-4-one

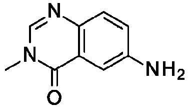

3-Methyl-6-nitroquinazolin-4-one (1.00 g, 4.87 mmol, Eq: 1.0) was suspended in methanol (25 mL) and AcOH (1 mL). Palladium on carbon (10 wt. % Pd, 100 mg, 940 µmol, Eq: 0.193) was added, and the reaction was stirred at rt under a balloon of hydrogen. After 14 h, the reaction was filtered through Celite (eluent MeOH), and concentrated in vacuo. The residue was concentrated twice from toluene, then dried further under high vacuum to afford the title compound as a dark brown solid (821 mg, 96% yield). The material was used without further purification, but could be further purified by recrystallization from boiling water, washing the resultant dark brown needles with cold water, isopropanol and diethyl ether. MS (ESI) m/z: 176.1 [M+H]⁺.

### Step 3: 3-chloro-6-fluoro-2-[(3-methyl-4-oxo-quinazolin-6-yl)amino]benzonitrile

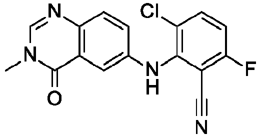

6-Amino-3-methylquinazolin-4-one (200 mg, 1.14 mmol, Eq: 1.0) and 3-chloro-2,6-difluorobenzonitrile (198 mg, 1.14 mmol, Eq: 1.0) were dissolved in DMSO (3 mL). Potassium tert-butoxide (141 mg, 1.26 mmol, Eq: 1.1) was added and the reaction was stirred at rt for 2 h. The reaction was diluted with water and extracted 2 × with EtOAc. The combined organic layers were washed with brine, dried (MgSO₄), filtered and concentrated in vacuo. Purification by flash chromatography (24 g, 0-5% MeOH in DCM) to give the title compound as a yellow solid (108 mg, 29 % yield). MS (ESI) m/z: 329.2 [M+H]⁺.

### Step 4: 6-azido-3-chloro-2-[(3-methyl-4-oxo-quinazolin-6-yl)amino]benzonitrile

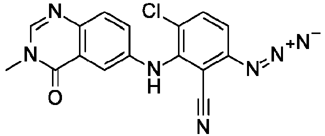

3-Chloro-6-fluoro-2-[(3-methyl-4-oxo-quinazolin-6-yl)amino]benzonitrile (320 mg, 973 µmol, Eq: 1.0), sodium azide (75.9 mg, 1.17 mmol, Eq: 1.2) and DMF dry (4 mL) were stirred at 120 °C for 1.5 h under a nitrogen atmosphere. The reaction mixture was cooled to rt and then diluted with water and extracted 2 × with EtOAc. The combined organic layers washed with brine, dried over Na₂SO₄, filtered and concentrated in vacuo to give the title compound as a brown solid (372 mg, 92% purity, quant.). MS (ESI) m/z: 352.2 [M+H]⁺.

### Step 5: 6-amino-3-chloro-2-[(3-methyl-4-oxo-quinazolin-6-yl)amino]benzonitrile

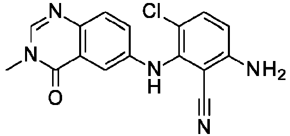

To a solution of 6-azido-3-chloro-2-[(3-methyl-4-oxo-quinazolin-6-yl)amino]benzonitrile (372 mg, 1.06 mmol, Eq: 1) in 2-propanol (10 mL) was added triethylamine (214 mg, 295 µL, 2.12 mmol, Eq: 2), 1,3-propanedithiol (80.1 mg, 74.9 µL, 740 µmol, Eq: 0.7) and sodium borohydride (40 mg, 1.06 mmol, Eq: 1). The reaction mixture was stirred at rt overnight, the concentrated in vacuo. EtOAc and citric acid (10% aq) were added to the residue, the phases were separated and the aqueous phase was extracted 2 × with EtOAc. The combined organic layers were washed with brine, dried over Na₂SO₄, filtered and evaporated to dryness to give the title compound as a yellow solid (345 mg, quant.). MS (ESI) m/z: 326.1 [M+H]⁺.

### Intermediate I9: (3R)-3-fluoropyrrolidine-1-sulfonamide

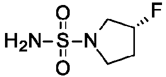

(*R*)-3-Fluoropyrrolidine hydrochloride (1.8 g, 14.3 mmol, Eq: 1.2) was added to a solution of sulfuric diamide (1.148 g, 11.9 mmol, Eq: 1.0) and triethylamine (2.42 g, 3.33 mL, 23.9 mmol, Eq: 2.0) in dioxane (10 mL). The reaction was stirred in a sealed tube at 115 °C for 15.5 h then cooled to rt and concentrated in vacuo. The residue was diluted with DCM, evaporated with silica gel to dryness and transferred to a column. Purification by flash chromatography (40 g silica, 80% EtOAc) gave the title compound as a white crystalline solid (1.82 g, 91% yield). MS (ESI) *m*/*z:* 169.1 [M+H]⁺.

### Intermediate I10: pyrrolidine-1-sulfonamide

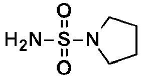

Pyrrolidine (1.78 g, 2.07 mL, 25 mmol, Eq: 1.2) was added to a solution of sulfuric diamide (2 g, 20.8 mmol, Eq: 1.0) in dioxane (20 mL). The reaction was stirred in a sealed tube at 115 °C for 15.5 h then cooled to rt and concentrated in vacuo. The residue was diluted with MeOH, evaporated with silica gel to dryness and transferred to a column. Purification by flash chromatography (40 g silica, 0-100% EtOAc in heptane) gave the title compound as a white solid (2.5 g, 80% yield). MS (ESI) *m*/*z:* 151.1 [M+H]⁺.

### Intermediate I11: cyclopentanesulfonamide

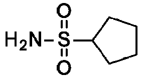

Cyclopentanesulfonyl chloride (675 mg, 619 µL, 4 mmol, Eq: 1.0) was added dropwise at rt to ammonium hydroxide solution (30-33% in water, 10.8 g, 12 mL, 92.5 mmol, Eq: 23.1). The reaction mixture was stirred overnight at rt. After 20.5 h, HCl (25% aq.) was added dropwise until the pH of the solution was 7. The reaction mixture was extracted 3 × with EtOAc, the combined organic layers were washed 1 × with brine, dried over Na₂SO₄, filtered and concentrated in vacuo. The residue was dried under high vacuum to give the title compound as a light brown solid (658 mg, 91% purity, 100% yield). ¹H NMR (300 MHz, DMSO-*d₆*) δ ppm 1.42 - 1.74 (m, 4 H) 1.76 - 1.95 (m, 4 H) 3.33 - 3.45 (m, 1 H) 6.69 (s, 2 H).

### Intermediate I12: (RS)-butane-2-sulfonamide

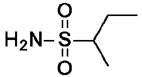

Following the procedure described for **I11**, the title compound was obtained from butane-2-sulfonyl chloride (626 mg, 4 mmol) as a light yellow oil (397 mg, 73% yield). ¹H NMR (300 MHz, CHLOROFORM-*d*) δ ppm 1.06 (t, *J*=7.5 Hz, 3 H) 1.41 (d, *J*=6.9 Hz, 3 H) 1.49 - 1.68 (m, 1 H) 1.98 - 2.27 (m, 1 H) 2.85 - 3.12 (m, 1 H) 4.44 (br s, 2 H).

### Intermediate I13: cyclohexanesulfonamide

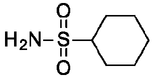

Following the procedure described for **I11**, the title compound was obtained from cyclohexanesulfonyl chloride (568 mg, 2.8 mmol) as a white solid (371 mg, 81% yield). ¹H NMR (300 MHz, DMSO-*d₆*) δ ppm 1.14 - 1.41 (m, 5 H) 1.55 - 1.69 (m, 1 H) 1.72 - 1.86 (m, 2 H) 2.06 (br d, *J*=10.7 Hz, 2 H) 2.63 - 2.89 (m, 1 H) 6.61 (s, 2 H).

### Intermediate I14: (R)-3-fluoropyrrolidine-1-sulfonyl chloride

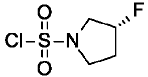

In a 500 mL 4-necked flask (purged with argon; equipped with thermometer and dripping funnel) (*R*)-3-fluoropyrrolidine hydrochloride (7 g, 55.7 mmol, Eq: 1.0) was combined with DCM (200 mL) under constant argon flow. DIPEA (21.6 g, 29.2 mL, 167 mmol, Eq: 3.0) was added to give a light yellow solution and the reaction mixture was cooled to -70 °C. Sulfuryl dichloride (15 g, 9.01 mL, 111 mmol, Eq: 2.0) in DCM (20 mL) was slowly added through a dropping funnel while maintaining the temperature at -70 °C. The reaction mixture was stirred at -70 °C for 1 h and was then allowed to come to rt over 1 h. The reaction mixture was poured into iced water in an Erlenmeyer flask. The mixture was transferred into a separating funnel and the phases were separated. The organic layer was washed with 1 N aq. HCl (100 mL). The aqueous layers were extracted with two more portions of DCM (80 mL each). The organic layers were combined, dried over Na₂SO₄, filtered and evaporated to dryness, then dried under high vacuum for 1 h to give the title compound as a brown solid (11.0 g, quant. yield) which was stored at 4 °C prior to use. ¹H NMR (300 MHz, CHLOROFORM-*d*) δ ppm 1.97 - 2.60 (m, 2 H) 3.53 - 3.92 (m, 4 H) 5.04 - 5.64 (m, 1 H)

### Intermediate I15: pyrrolidine-1-sulfonyl chloride

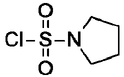

Following the procedure described for Intermediate **I14**, the title compound was obtained from pyrrolidine (2.17 g, 2.5 mL, 30.4 mmol, Eq: 1.0) and sulfuryl dichloride (8.22 g, 4.92 mL, 60.9 mmol, Eq: 2.0) as a brown liquid (4.4 g, 85% yield). ¹H NMR (300 MHz, CHLOROFORM-*d*) δ ppm 1.90 - 2.14 (m, 4 H) 3.36 - 3.63 (m, 4 H).

### Example 1: 6-[2-cyano-3-[[ethyl(methyl)sulfamoyl]amino]-6-fluoro-phenoxy]-3-methyl-4-oxo-quinazoline

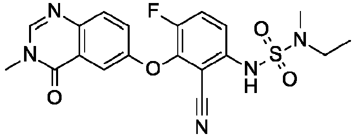

[Methyl(sulfamoyl)amino]ethane (185 mg, 1.34 mmol, Eq: 2.1) was dissolved in NMP (8 mL). At 0 °C NaH (60% in mineral oil, 61.3 mg, 1.4 mmol, Eq: 2.2) was added, the cooling bath was removed and the reaction mixture was stirred at 50 °C for 30 min. The reaction mixture was cooled to 0 °C, then a solution of **I2** (200 mg, 638 µmol, Eq: 1.0) in NMP (2 mL) was added. The reaction mixture was stirred at 125 °C for 1 h. The reaction was cooled to rt, and the reaction mixture was taken up in 10 mL 0.1 M aq. NaOH, ice and EtOAc. The aq. layer was separated and extracted again with EtOAc. The aq. layer was acidified with 2 M aq. HCl to pH 4 and extracted 2 × with EtOAc, the combined org. layers were washed 3 × with water, 1 × with brine, then dried over Na₂SO₄, filtrated and evaporated. The residue was diluted with DCM, evaporated with silica gel to dryness and transferred to a column. Purification by flash chromatography (40 g silica, 0-100% EtOAc in heptane) gave the title compound as a white solid (161 mg, 97% purity, 57% yield). MS (ESI) *m*/*z:* 432.2 [M+H]⁺.

### Example 2: 6-[6-chloro-2-cyano-3-[[ethyl(methyl)sulfamoyl]amino]phenoxy]-3-methyl-4-oxo-quinazoline

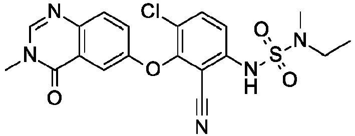

**I4** (45 mg, 138 µmol, Eq: 1.0) was dissolved in DCM (275 µL) in a sealable tube. Pyridine (490 mg, 501 µL, 6.2 mmol, Eq: 45), DMAP (1.68 mg, 13.8 µmol, Eq: 0.1) and N-ethyl-N-methylsulfamoyl chloride (65 mg, 413 µmol, Eq: 3.0) were added at rt. The tube was sealed and the reaction was heated at 80 °C. After 24 h, the reaction was cooled to rt, diluted with DCM (20 mL) washed with 10% aq. citric acid (2 × 20 mL), water (20 mL) and brine (20 mL), dried (Na₂SO₄), filtered and concentrated in vacuo. The crude mixture was dry-loaded onto Isolute and purified by flash chromatography (50-100% EtOAc in heptane). The resultant material was further purified by reversed phase HPLC to give the title compound as a colourless lyophilised solid (20 mg, 100% purity, 32% yield). MS (ESI) *m*/*z:* 448.2, 450.1 [M+H]⁺.

### Example 3: (3R)-N-[4-chloro-2-cyano-3-(3-methyl-4-oxo-quinazolin-6-yl)oxy-phenyl]-3-fluoro-pyrrolidine-1-sulfonamide

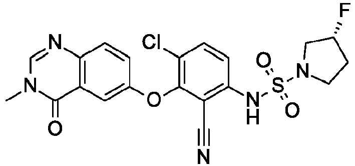

Following the procedure described for example 2, the title compound was obtained from **I4** (55 mg, 168 µmol, Eq: 1.0) and **I14** (129 mg, 688 µmol, Eq: 4.1) as an off-white lyophilised solid following reversed phase HPLC purification (7 mg, 98% purity, 8.5% yield). MS (ESI) *m*/*z:* 478.0759 [M+H]⁺.

### Example 4: N-[4-chloro-2-cyano-3-(3-methyl-4-oxo-quinazolin-6-yl)oxy-phenyl]pyrrolidine-1-sulfonamide

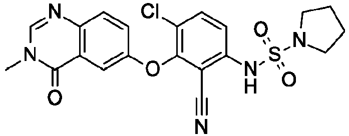

Following the procedure described for Example **2,** the title compound was obtained from **I4** (100 mg, 306 µmol, Eq: 1.0) and **I15** (156 mg, 918 µmol, Eq: 3.0) as an off-white solid following flash chromatography (1-6% MeOH in DCM) (25 mg, 98% purity, 17% yield). MS (ESI) *m*/*z:* 460.2, 462.2 [M+H]⁺.

### Example 5: 6-[2-cyano-3-[[ethyl(methyl)sulfamoyl]amino]phenoxy]-3-methyl-4-oxo-quinazoline

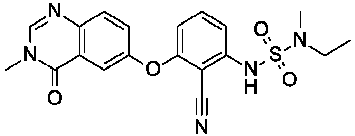

[Methyl(sulfamoyl)amino]ethane (98.3 mg, 711 µmol, Eq: 2.1) was disolved in DMF (4 mL). At 0 °C NaH (60% in mineral oil, 32.5 mg, 745 µmol, Eq: 2.2) was added, the cooling bath was removed and the reaction mixture was stirred at 50 °C for 20 min. The reaction mixture was cooled to 0 °C, then **I3** (100 mg, 339 µmol, Eq: 1.0) was added. The reaction mixture was stirred at 100 °C for 21 h, then concentrated in vacuo. The residue was suspended in sat. aq. NH₄Cl (40 mL), and extracted with DCM (3 × 40 mL). The combined organic layers were washed with brine (100 mL), dried (Na₂SO₄), filtered and concentrated in vacuo. Purification by SFC gave the title compound as a colourless solid (60 mg, 98% purity, 42% yield). MS (ESI) *m*/*z:* 414.2 [M+H]⁺.

### Example 6: N-[2-cyano-3-(3-methyl-4-oxo-quinazolin-6-yl)oxy-phenyl]pyrrolidine-1-sulfonamide

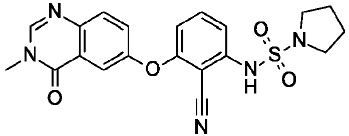

Following the procedure described for Example **5,** the title compound was obtained from **I3** (100 mg, 339 µmol, Eq: 1.0) and **I10** (107 mg, 711 µmol, Eq: 2.1) as a colourless solid following SFC purification (48 mg, 100% purity, 33% yield). MS (ESI) *m*/*z:* 426.3 [M+H]⁺.

### Example 7: (3R)-N-[2-cyano-3-(3-methyl-4-oxo-quinazolin-6-yl)oxy-phenyl]-3-fluoro-pyrrolidine-1-sulfonamide

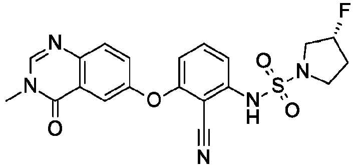

Following the procedure described for Example 5, the title compound was obtained from **I3** (100 mg, 339 µmol, Eq: 1.0) and **I9** (120 mg, 711 µmol, Eq: 2.1) as a colourless solid following SFC purification (80 mg, 93% purity, 50% yield). MS (ESI) *m*/*z:* 444.2 [M+H]⁺.

### Example 8: N-[2-cyano-3-(3-methyl-4-oxo-quinazolin-6-yl)oxyphenyl]cyclopentanesulfonamide

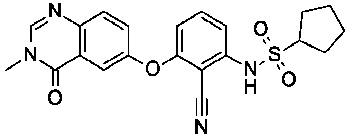

Following the procedure described for Example 5, the title compound was obtained from **I3** (75 mg, 254 µmol, Eq: 1.0) and **I11** (79.6 mg, 533 µmol, Eq: 2.1) as a colourless solid following reversed phase HPLC purification (63 mg, 100% purity, 58% yield). MS (ESI) *m*/*z:* 425.3 [M+H]⁺.

### Example 9: N-[2-cyano-3-(3-methyl-4-oxo-quinazolin-6-yl)oxy-phenylicyclohexanesulfonamide

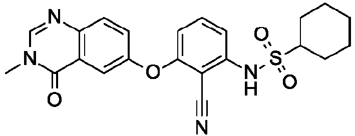

Following the procedure described for Example 5, the title compound was obtained from **I3**(75 mg, 254 µmol, Eq: 1.0) and **I13** (87.1 mg, 533 µmol, Eq: 2.1) as a colourless solid following reversed phase HPLC purification (71 mg, 98% purity, 63% yield). MS (ESI) *m*/*z:* 439.3 [M+H]⁺.

### Example 10: (RS)-N-[2-cyano-3-(3-methyl-4-oxo-quinazolin-6-yl)oxy-phenyl]butane-2-sulfonamide

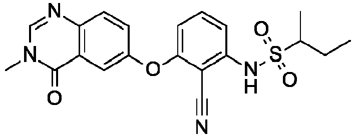

Following the procedure described for Example 5, the title compound was obtained from **I3** (75 mg, 254 µmol, Eq: 1.0) and **I12** (73.2 mg, 533 µmol, Eq: 2.1) as a colourless solid following reversed phase HPLC purification (51 mg, 100% purity, 49% yield). MS (ESI) *m*/*z:* 413.3 [M+H]⁺.

### Example 11: 6-[2-cyano-3-[[ethyl(methyl)sulfamoyl]amino]anilino]-3-methyl-4-oxo-quinazoline

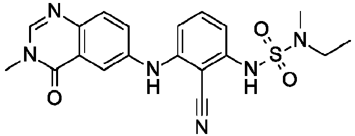

6-Bromo-3-methylquinazolin-4(3H)-one (21 mg, 86.1 µmol, Eq: 1.0) and **I5** (21.9 mg, 86.1 µmol, Eq: 1.0) were dissolved in dioxane (1.6 mL) then Cs₂CO₃ (85 mg, 258 µmol, Eq: 3.0) was added. The reaction mixture was flushed with argon, then BippyPhos (2.7 mg, 5.16 µmol, Eq: 0.06) and tris(dibenzylideneacetone)dipalladium (0) chloroform adduct (2.73 mg, 2.58 µmol, Eq: 0.03) were added. The reaction was flushed with argon again, and the vial was closed. The reaction mixture was heated to 110 °C and stirred for 1 h. The reaction mixture was taken up in 15 mL 2-methyl-THF and ice, and washed with 4 mL 1% aq. citric acid. The aqueous layer was back-extracted with 1 × 15 mL 2-methyl-THF. The organic layers were combined, washed with brine, dried over Na₂SO₄ and concentrated in vacuo. The residue was diluted with EtOAc and transferred to a column. Purification by flash chromatography (40 g silica, 100% EtOAc) followed by preparative reversed phase HPLC gave the title compound as a white solid (13 mg, 94% purity, 53% yield). MS (ESI) *m*/*z:* 413.2 [M+H]⁺.

### Example 12: (3R)-N-[2-cyano-3-[(3-methyl-4-oxo-quinazolin-6-yl)amino]phenyl]-3-fluoro-pyrrolidine-1-sulfonamide

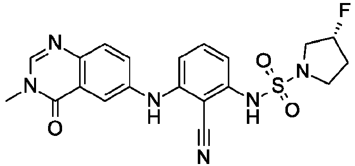

**I7** (20.5 mg, 70.4 µmol, Eq: 1.0) was dissolved in DCM (150 µL). At rt, pyridine (256 mg, 260 µL, 3.24 mmol, Eq: 46), DMAP (877 µg, 7.04 µmol, Eq: 0.1) and **I14** (39.6 mg, 211 µmol, Eq: 3.0) were added. The reaction mixture was stirred at 75 °C for 21.5 h. The reaction mixture was taken up in 2-methyl-THF, ice and 1% aq. citric acid. The aqueous layer was back-extracted 2 × with 2-methyl-THF. The organic layers were combined, washed with brine, dried over Na₂SO₄ and concentrated in vacuo. The residue was diluted with DCM, evaporated with silica gel to dryness and transferred to a column. Purification by flash chromatography (25 g silica, 100% EtOAc) gave the title compound as a light yellow solid (5.1 mg, 95% purity, 16% yield). MS (ESI) *m*/*z:* 443.2 [M+H]⁺.

### Example 13: 6-[6-chloro-2-cyano-3-[[ethyl(methyl)sulfamoyl]amino]anilino]-3-methyl-4-oxo-quinazoline

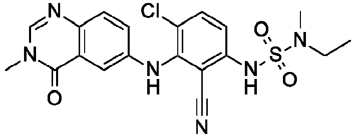

**I8** (75 mg, 230 µmol, Eq: 1) was dissolved in pyridine (1 mL) and DCM (1 mL) in a vial. Ethyl(methyl)sulfamoyl chloride (90.7 mg, 576 µmol, Eq: 2.5) and DMAP (1.41 mg, 11.5 µmol, Eq: 0.05) in DCM (1 mL) were added to the reaction mixture, and the vial was sealed. The reaction mixture was stirred for two days at 60 °C. The reaction mixture was quenched with water, diluted with DCM and washed 2 × with citric acid (10% aq.). The aqueous layers were extracted 2 × with DCM. The organic layers were combined, dried over Na₂SO₄, filtered and concentrated in vacuo. Purification by flash chromatography (12 g silica, 0-5% MeOH in DCM) gave the title compound as an orange solid (25 mg, 95% purity, 24% yield). MS (ESI) *m*/*z:* 447.1 [M+H]⁺.

### Example 14: N-[4-chloro-2-cyano-3-[(3-methyl-4-oxo-quinazolin-6-yl)amino]phenyl]pyrrolidine-1-sulfonamide

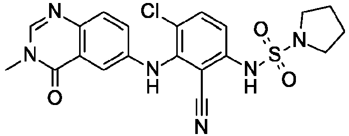

Following the procedure described for Example **13,** the title compound was obtained from **I8** (75 mg, 230 µmol, Eq: 1.0) and **I15** (122 mg, 576 µmol, Eq: 2.5) as a white solid (10 mg, 100% purity, 9% yield) after preparative reverse phase HPLC purification. MS (ESI) *m*/*z:* 459.2 [M+H]⁺.

### Example 15: N-[2-cyano-4-fluoro-3-(3-methyl-4-oxo-quinazolin-6-yl)oxy-phenyl]pyrrolidine-1-sulfonamide

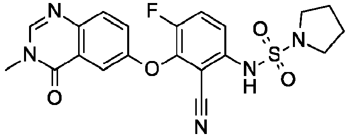

**I10** (50.3 mg, 335 µmol, Eq: 2.1) and cesium carbonate (114 mg, 351 µmol, Eq: 2.2) were combined in a heat-dried reaction tube. The tube was set under argon atmosphere and DMF (456 µl) added. The mixture was stirred for 30 minutes at 50 °C. Then, the reaction was allowed to cool down to room temperature. **I2** (50 mg, 160 µmol, Eq: 1.0) in DMF (1.14 ml) was added. The tube was sealed and the reaction was stirred at 100 °C for 16 h. The mixture was taken up in sat. aq. NH₄Cl (10 mL) and EtOAc (10 mL). The phases were separated, and the aqueous layer was extracted further with 3 × 10 mL EtOAc. The combined organic layers were washed with water (30 mL) and brine (30 mL), dried (Na₂SO₄), filtered and concentrated in vacuo. The residue was diluted with DCM and transferred to a column. Purification by flash chromatography (12 g silica, 0-3% MeOH/DCM) gave the title compound (40 mg, 100% purity, 56.5% yield) as a colourless solid. MS (ESI) *m*/*z:* 444.2 [M+H]⁺.

### Example 16: N-[2-cyano-4-fluoro-3-(3-methyl-4-oxo-quinazolin-6-yl)oxyphenyl]cyclopentanesulfonamide

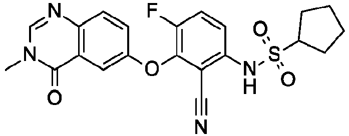

Following the procedure described for Example **15,** the title compound was obtained from **I11** (50 mg, 335 µmol, Eq: 2.1) and **I2** (50 mg, 160 µmol, Eq: 1.0) as a colourless solid (43 mg, 100% purity, 61% yield) after flash chromatography and SFC purification. MS (ESI) *m*/*z:* 443.2 [M+H]⁺.

### Example 17: 6-[2-cyano-3-(dimethylsulfamoylamino)-6-fluoro-phenoxy]-3-methyl-4-oxo-quinazoline

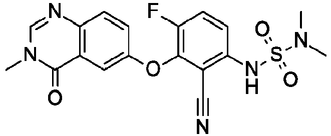

Following the procedure described for Example **15,** the title compound was obtained from *N,N-*dimethylsulfamide (41.6 mg, 335 µmol, Eq: 2.1) and **I2** (50 mg, 160 µmol, Eq: 1.0) as a white foam (34 mg, 100% purity, 51% yield) after flash chromatography. MS (ESI) *m*/*z:* 418.2 [M+H]⁺.

## Claims

1. A compound of formula (I) 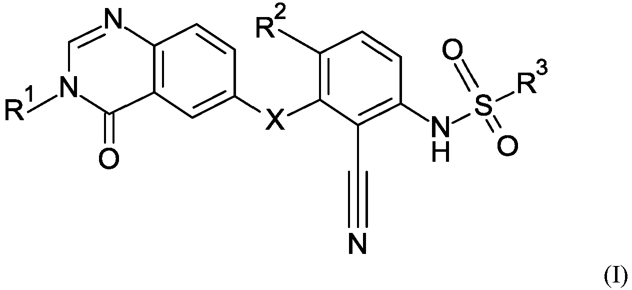 wherein
R¹ is C₁₋₆-alkyl;
X is selected from
i) -NH-, and
ii) -O-;
R² is selected from
iii) H,
iv) cyano, and
v) halogen;
R³ is selected from
vi) NR⁴R⁵, and
vii) CHR⁶R⁷;
R⁴ is selected from
viii) C₁₋₆-alkyl,
ix) C₃₋₈-cycloalkyl, and
x) C₃₋₈-cycloalkyl-C₁₋₆-alkyl;
R⁵ is selected from
xi) C₁₋₆-alkyl,
xii) C₃₋₈-cycloalkyl, and
xiii) C₃₋₈-cycloalkyl-C₁₋₆-alkyl;
or R⁴ and R⁵ together with the nitrogen atom to which they are attached form an heterocycloalkyl optionally substituted with R⁸, wherein the heterocycloalkyl is selected from pyrrolidinyl and piperidinyl;
R⁶ is selected from
xiv) C₁₋₆-alkyl,
xv) C₃₋₈-cycloalkyl, and
xvi) C₃₋₈-cycloalkyl-C₁₋₆-alkyl;
R⁷ is selected from
xvii) C₁₋₆-alkyl,
xviii) C₃₋₈-cycloalkyl, and
xix) C₃₋₈-cycloalkyl-C₁₋₆-alkyl;
or R⁶ and R⁷ together with the carbon atom to which they are attached form a C₃₋₈-cycloalkyl optionally substituted with R⁸;
R⁸ is halogen;
with the proviso that N-[2-cyano-4-fluoro-3-(3-methyl-4-oxo-quinazolin-6-yl)oxy-phenyl]-3-fluoro-pyrrolidine-1-sulfonamide is excluded
or a pharmaceutically acceptable salt thereof.

2. A compound according to claim 1, wherein
R¹ is C₁₋₆-alkyl;
X is selected from
i) -NH-, and
ii) -O-;
R² is selected from
iii) H, and
iv) halogen;
R³ is selected from
v) NR⁴R⁵, and
vi) CHR⁶R⁷;
R⁴ is selected from
vii) C₁₋₆-alkyl,
viii) C₃₋₈-cycloalkyl, and
ix) C₃₋₈-cycloalkyl-C₁₋₆-alkyl;
R⁵ is selected from
x) C₁₋₆-alkyl,
xi) C₃₋₈-cycloalkyl, and
xii) C₃₋₈-cycloalkyl-C₁₋₆-alkyl;
or R⁴ and R⁵ together with the nitrogen atom to which they are attached form an heterocycloalkyl optionally substituted with R⁸, wherein the heterocycloalkyl is selected from pyrrolidinyl and piperidinyl;
R⁶ is selected from
xiii) C₁₋₆-alkyl,
xiv) C₃₋₈-cycloalkyl, and
xv) C₃₋₈-cycloalkyl-C₁₋₆-alkyl;
R⁷ is selected from
xvi) C₁₋₆-alkyl,
xvii) C₃₋₈-cycloalkyl, and
xviii) C₃₋₈-cycloalkyl-C₁₋₆-alkyl;
or R⁶ and R⁷ together with the carbon atom to which they are attached form a cyclopentyl or a cyclohexyl ring optionally substituted with R⁸;
R⁸ is halogen;
with the proviso that (3R)-N-[2-cyano-4-fluoro-3-(3-methyl-4-oxo-quinazolin-6-yl)oxyphenyl]-3-fluoro-pyrrolidine-1-sulfonamide is excluded
or a pharmaceutically acceptable salt thereof.

3. A compound according to claim 1 or 2, wherein
R¹ is methyl;
X is selected from
i) -NH-, and
ii) -O-;
R² is selected from
iii) H,
iv) chloro, and
v) fluoro;
R³ is selected from
vi) NR⁴R⁵, and
vii) CHR⁶R⁷;
R⁴ is selected from
viii) methyl,
ix) ethyl,
x) propyl,
xi) cyclopropyl, and
xii) cyclopropylmethyl;
R⁵ is selected from
xiii) methyl,
xiv) ethyl,
xv) propyl,
xvi) cyclopropyl, and
xvii) cyclopropylmethyl;
or R⁴ and R⁵ together with the nitrogen atom to which they are attached form an heterocycloalkyl optionally substituted with R⁸, wherein the heterocycloalkyl is selected from pyrrolidinyl and piperidinyl;
R⁶ is selected from
xviii) methyl,
xix) ethyl,
xx) propyl,
xxi) cyclopropyl, and
xxii) cyclopropylmethyl;
R⁷ is selected from
xxiii) methyl,
xxiv) ethyl,
xxv) propyl,
xxvi) cyclopropyl, and
xxvii) cyclopropylmethyl;
or R⁶ and R⁷ together with the carbon atom to which they are attached form a cyclopentyl or a cyclohexyl ring optionally substituted with R⁸;
R⁸ is fluoro;
with the proviso that (3R)-N-[2-cyano-4-fluoro-3-(3-methyl-4-oxo-quinazolin-6-yl)oxyphenyl]-3-fluoro-pyrrolidine-1-sulfonamide is excluded
or a pharmaceutically acceptable salt thereof.

4. A compound according to any one of claims 1 to 3, wherein R² is selected from
i) H,
ii) chloro, and
iii) fluoro.

5. A compound according to any one of claims 1 to 4, wherein R⁴ is methyl.

6. A compound according to any one of claims 1 to 5, wherein R⁵ is ethyl.

7. A compound according to any one of claims 1 to 6, wherein R⁴ and R⁵ together with the nitrogen atom to which they are attached form an heterocycloalkyl optionally substituted with R⁸, wherein the heterocycloalkyl is selected from pyrrolidinyl and piperidinyl.

8. A compound according to claim 7, wherein R⁴ and R⁵ together with the nitrogen atom to which they are attached form an unsubstituted heterocycloalkyl, wherein the heterocycloalkyl is pyrrolidinyl.

9. A compound according to any one of claims 1 to 8, wherein R⁶ and R⁷ are independently selected from
i) methyl,
ii) ethyl,
iii) propyl,
iv) cyclopropyl, and
v) cyclopropylmethyl.

10. A compound according to any one of claims 1 to 8, wherein R⁶ and R⁷ together with the nitrogen atom to which they are attached form a cyclopentyl or a cyclohexyl ring.

11. A compound according to any one of claims 1 to 10, wherein R⁸ is fluoro.

12. A compound according to any one of claims 1 to 11, wherein the compound is selected from
6-[2-cyano-3-[[ethyl(methyl)sulfamoyl]amino]-6-fluoro-phenoxy]-3-methyl-4-oxo-quinazoline;
6-[6-chloro-2-cyano-3-[[ethyl(methyl)sulfamoyl]amino]phenoxy]-3-methyl-4-oxo-quinazoline;
(3R)-N-[4-chloro-2-cyano-3-(3-methyl-4-oxo-quinazolin-6-yl)oxy-phenyl]-3-fluoro-pyrrolidine-1-sulfonamide;
N-[4-chloro-2-cyano-3-(3-methyl-4-oxo-quinazolin-6-yl)oxy-phenyl]pyrrolidine-1-sulfonamide;
6-[2-cyano-3-[[ethyl(methyl)sulfamoyl]amino]phenoxy]-3-methyl-4-oxo-quinazoline;
N-[2-cyano-3-(3-methyl-4-oxo-quinazolin-6-yl)oxy-phenyl]pyrrolidine-1-sulfonamide;
(3R)-N-[2-cyano-3-(3-methyl-4-oxo-quinazolin-6-yl)oxy-phenyl]-3-fluoro-pyrrolidine-1-sulfonamide;
N-[2-cyano-3-(3-methyl-4-oxo-quinazolin-6-yl)oxy-phenyl]cyclopentanesulfonamide;
N-[2-cyano-3-(3-methyl-4-oxo-quinazolin-6-yl)oxy-phenyl]cyclohexanesulfonamide;
N-[2-cyano-3-(3-methyl-4-oxo-quinazolin-6-yl)oxy-phenyl]butane-2-sulfonamide;
6-[2-cyano-3-[[ethyl(methyl)sulfamoyl]amino]anilino]-3-methyl-4-oxo-quinazoline;
(3R)-N-[2-cyano-3-[(3-methyl-4-oxo-quinazolin-6-yl)amino]phenyl]-3-fluoro-pyrrolidine-1-sulfonamide;
6-[6-chloro-2-cyano-3-[[ethyl(methyl)sulfamoyl]amino]anilino]-3-methyl-4-oxo-quinazoline;
N-[4-chloro-2-cyano-3-[(3-methyl-4-oxo-quinazolin-6-yl)amino]phenyl]pyrrolidine-1-sulfonamide;
N-[2-cyano-4-fluoro-3-(3-methyl-4-oxo-quinazolin-6-yl)oxy-phenyl]pyrrolidine-1-sulfonamide;
N-[2-cyano-4-fluoro-3-(3-methyl-4-oxo-quinazolin-6-yl)oxyphenyl]cyclopentanesulfonamide; and
6-[2-cyano-3-(dimethylsulfamoylamino)-6-fluoro-phenoxy]-3-methyl-4-oxo-quinazoline;
or a pharmaceutically acceptable salt thereof.

13. A compound according to claim 12, wherein the compound is selected from
6-[2-cyano-3-[[ethyl(methyl)sulfamoyl]amino]-6-fluoro-phenoxy]-3-methyl-4-oxo-quinazoline;
6-[6-chloro-2-cyano-3-[[ethyl(methyl)sulfamoyl]amino]phenoxy]-3-methyl-4-oxo-quinazoline;
N-[4-chloro-2-cyano-3-(3-methyl-4-oxo-quinazolin-6-yl)oxy-phenyl]pyrrolidine-1-sulfonamide;
6-[2-cyano-3-[[ethyl(methyl)sulfamoyl]amino]phenoxy]-3-methyl-4-oxo-quinazoline;
N-[2-cyano-3-(3-methyl-4-oxo-quinazolin-6-yl)oxy-phenyl]pyrrolidine-1-sulfonamide;
N-[2-cyano-3-(3-methyl-4-oxo-quinazolin-6-yl)oxy-phenyl]cyclopentanesulfonamide;
N-[2-cyano-3-(3-methyl-4-oxo-quinazolin-6-yl)oxy-phenyl]cyclohexanesulfonamide;
N-[2-cyano-3-(3-methyl-4-oxo-quinazolin-6-yl)oxy-phenyl]butane-2-sulfonamide;
6-[2-cyano-3-[[ethyl(methyl)sulfamoyl]amino]anilino]-3-methyl-4-oxo-quinazoline;
6-[6-chloro-2-cyano-3-[[ethyl(methyl)sulfamoyl]amino]anilino]-3-methyl-4-oxo-quinazoline;
N-[4-chloro-2-cyano-3-[(3-methyl-4-oxo-quinazolin-6-yl)amino]phenyl]pyrrolidine-1-sulfonamide;
N-[2-cyano-4-fluoro-3-(3-methyl-4-oxo-quinazolin-6-yl)oxy-phenyl]pyrrolidine-1-sulfonamide;
N-[2-cyano-4-fluoro-3-(3-methyl-4-oxo-quinazolin-6-yl)oxyphenyl]cyclopentanesulfonamide; and
6-[2-cyano-3-(dimethylsulfamoylamino)-6-fluoro-phenoxy]-3-methyl-4-oxo-quinazoline;
or a pharmaceutically acceptable salt thereof.

14. A compound according to any one of claims 1 to 13 for use as therapeutically active substance.

15. A pharmaceutical composition comprising a compound according to any one of claims 1 to 13 and a therapeutically inert carrier.

16. A compound according to any one of claims 1 to 13 for use in the treatment or prophylaxis of thyroid cancer, colorectal cancer, brain cancer, melanoma or non-small cell lung cancer.

## Patentansprüche

1. Verbindung der Formel (I) 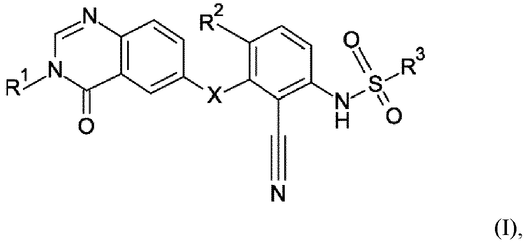 wobei
R¹ C₁₋₆-Alkyl ist;
X aus
i) -NH- und
ii) -O-
ausgewählt ist;
R² aus
iii) H,
iv) Cyano und
v) Halogen
ausgewählt ist;
R³ aus
vi) NR⁴R⁵ und
vii) CHR⁶R⁷
ausgewählt ist;
R⁴ aus
viii) C₁₋₆-Alkyl,
ix) C₃₋₈-Cycloalkyl und
x) C₃₋₈-Cycloalkyl-C₁₋₆-alkyl
ausgewählt ist;
R⁵ aus
xi) C₁₋₆-Alkyl,
xii) C₃₋₈-Cycloalkyl und
xiii) C₃₋₈-Cycloalkyl-C₁₋₆-alkyl
ausgewählt ist;
oder R⁴ und R⁵ zusammen mit dem Stickstoffatom, an das sie gebunden sind, ein Heterocycloalkyl bilden, das gegebenenfalls mit R⁸ substituiert ist, wobei das Heterocycloalkyl aus Pyrrolidinyl und Piperidinyl ausgewählt ist;
R⁶ aus
xiv) C₁₋₆-Alkyl,
xv) C₃₋₈-Cycloalkyl und
xvi) C₃₋₈-Cycloalkyl-C₁₋₆-alkyl
ausgewählt ist;
R⁷ aus
xvii) C₁₋₆-Alkyl,
xviii) C₃₋₈-Cycloalkyl und
xix) C₃₋₈-Cycloalkyl-C₁₋₆-alkyl
ausgewählt ist;
oder R⁶ und R⁷ zusammen mit dem Kohlenstoffatom, an das sie gebunden sind, ein C₃₋₈-Cycloalkyl bilden, das gegebenenfalls mit R⁸ substituiert ist;
R⁸ Halogen ist;
mit der Maßgabe, dass N-[2-Cyano-4-fluor-3-(3-methyl-4-oxochinazolin-6-yl)oxyphenyl]-3-fluorpyrrolidin-1-sulfonamid ausgeschlossen ist;
oder ein pharmazeutisch unbedenkliches Salz davon.

2. Verbindung nach Anspruch 1, wobei
R¹ C₁₋₆-Alkyl ist;
X aus
i) -NH- und
ii) -O-
ausgewählt ist;
R² aus
iii) H und
iv) Halogen
ausgewählt ist;
R³ aus
v) NR⁴R⁵ und
vi) CHR⁶R⁷
ausgewählt ist;
R⁴ aus
vii) C₁₋₆-Alkyl,
viii) C₃₋₈-Cycloalkyl und
ix) C₃₋₈-Cycloalkyl-C₁₋₆-alkyl
ausgewählt ist;
R⁵ aus
x) C₁₋₆-Alkyl,
xi) C₃₋₈-Cycloalkyl und
xii) C₃₋₈-Cycloalkyl-C₁₋₆-alkyl
ausgewählt ist;
oder R⁴ und R⁵ zusammen mit dem Stickstoffatom, an das sie gebunden sind, ein Heterocycloalkyl bilden, das gegebenenfalls mit R⁸ substituiert ist, wobei das Heterocycloalkyl aus Pyrrolidinyl und Piperidinyl ausgewählt ist;
R⁶ aus
xiii) C₁₋₆-Alkyl,
xiv) C₃₋₈-Cycloalkyl und
xv) C₃₋₈-Cycloalkyl-C₁₋₆-alkyl
ausgewählt ist;
R⁷ aus
xvi) C₁₋₆-Alkyl,
xvii) C₃₋₈-Cycloalkyl und
xviii) C₃₋₈-Cycloalkyl-C₁₋₆-alkyl
ausgewählt ist;
oder R⁶ und R⁷ zusammen mit dem Kohlenstoffatom, an das sie gebunden sind, einen Cyclopentyl- oder einen Cyclohexylring bilden, der gegebenenfalls mit R⁸ substituiert ist; R⁸ Halogen ist;
mit der Maßgabe, dass (3R)-N-[2-Cyano-4-fluor-3-(3-methyl-4-oxochinazolin-6-yl)oxyphenyl]-3-fluorpyrrolidin-1-sulfonamid ausgeschlossen ist;
oder ein pharmazeutisch unbedenkliches Salz davon.

3. Verbindung nach Anspruch 1 oder 2, wobei
R¹ Methyl ist;
X aus
i) -NH- und
ii) -O-
ausgewählt ist;
R² aus
iii) H,
iv) Chlor und
v) Fluor
ausgewählt ist;
R³ aus
vi) NR⁴R⁵ und
vii) CHR⁶R⁷
ausgewählt ist;
R⁴ aus
viii) Methyl,
ix) Ethyl,
x) Propyl,
xi) Cyclopropyl und
xii) Cyclopropylmethyl
ausgewählt ist;
R⁵ aus
xiii) Methyl,
xiv) Ethyl,
xv) Propyl,
xvi) Cyclopropyl und
xvii) Cyclopropylmethyl
ausgewählt ist;
oder R⁴ und R⁵ zusammen mit dem Stickstoffatom, an das sie gebunden sind, ein Heterocycloalkyl bilden, das gegebenenfalls mit R⁸ substituiert ist, wobei das Heterocycloalkyl aus Pyrrolidinyl und Piperidinyl ausgewählt ist;
R⁶ aus
xviii) Methyl,
xix) Ethyl,
xx) Propyl,
xxi) Cyclopropyl und
xxii) Cyclopropylmethyl
ausgewählt ist;
R⁷ aus
xxiii) Methyl,
xxiv) Ethyl,
xxv) Propyl,
xxvi) Cyclopropyl und
xxvii) Cyclopropylmethyl
ausgewählt ist;
oder R⁶ und R⁷ zusammen mit dem Kohlenstoffatom, an das sie gebunden sind, einen Cyclopentyl- oder einen Cyclohexylring bilden, der gegebenenfalls mit R⁸ substituiert ist; R⁸ Fluor ist;
mit der Maßgabe, dass (3R)-N-[2-Cyano-4-fluor-3-(3-methyl-4-oxochinazolin-6-yl)oxyphenyl]-3-fluorpyrrolidin-1-sulfonamid ausgeschlossen ist;
oder ein pharmazeutisch unbedenkliches Salz davon.

4. Verbindung nach einem der Ansprüche 1 bis 3, wobei R² aus
i) H,
ii) Chlor und
iii) Fluor
ausgewählt ist.

5. Verbindung nach einem der Ansprüche 1 bis 4, wobei R⁴ Methyl ist.

6. Verbindung nach einem der Ansprüche 1 bis 5, wobei R⁵ Ethyl ist.

7. Verbindung nach einem der Ansprüche 1 bis 6, wobei R⁴ und R⁵ zusammen mit dem Stickstoffatom, an das sie gebunden sind, ein Heterocycloalkyl bilden, das gegebenenfalls mit R⁸ substituiert ist, wobei das Heterocycloalkyl aus Pyrrolidinyl und Piperidinyl ausgewählt ist.

8. Verbindung nach Anspruch 7, wobei R⁴ und R⁵ zusammen mit dem Stickstoffatom, an das sie gebunden sind, ein unsubstituiertes Heterocycloalkyl bilden, wobei das Heterocycloalkyl Pyrrolidinyl ist.

9. Verbindung nach einem der Ansprüche 1 bis 8, wobei R⁶ und R⁷ unabhängig voneinander aus
i) Methyl,
ii) Ethyl,
iii) Propyl,
iv) Cyclopropyl und
v) Cyclopropylmethyl
ausgewählt sind.

10. Verbindung nach einem der Ansprüche 1 bis 8, wobei R⁶ und R⁷ zusammen mit dem Stickstoffatom, an das sie gebunden sind, einen Cyclopentyl- oder einen Cyclohexylring bilden.

11. Verbindung nach einem der Ansprüche 1 bis 10, wobei R⁸ Fluor ist.

12. Verbindung nach einem der Ansprüche 1 bis 11, wobei die Verbindung aus Folgenden ausgewählt ist
6-[2-Cyano-3-[[ethyl(methyl)sulfamoyl]amino]-6-fluorphenoxy]-3-methyl-4-oxochinazolin;
6-[6-Chlor-2-cyano-3-[[ethyl(methyl)sulfamoyl]amino]phenoxy]-3-methyl-4-oxochinazolin;
(3R)-N-[4-Chlor-2-cyano-3-(3-methyl-4-oxochinazolin-6-yl)oxyphenyl]-3-fluorpyrrolidin-1-sulfonamid;
N-[4-Chlor-2-cyano-3-(3-methyl-4-oxochinazolin-6-yl)oxyphenyl]pyrrolidin-1-sulfonamid;
6-[2-Cyano-3-[[ethyl(methyl)sulfamoyl]amino]phenoxy]-3-methyl-4-oxochinazolin;
N-[2-Cyano-3-(3-methyl-4-oxochinazolin-6-yl)oxyphenyl]pyrrolidin-1-sulfonamid;
(3R)-N-[2-Cyano-3-(3-methyl-4-oxochinazolin-6-yl)oxyphenyl]-3-fluorpyrrolidin-1-sulfonamid;
N-[2-Cyano-3-(3-methyl-4-oxochinazolin-6-yl)oxyphenyl]cyclopentansulfonamid;
N-[2-Cyano-3-(3-methyl-4-oxochinazolin-6-yl)oxyphenyl]cyclohexansulfonamid;
N-[2-Cyano-3-(3-methyl-4-oxochinazolin-6-yl)oxyphenyl]butan-2-sulfonamid;
6-[2-Cyano-3-[[ethyl(methyl)sulfamoyl]amino]anilino]-3-methyl-4-oxochinazolin;
(3R)-N-[2-Cyano-3-[(3-methyl-4-oxochinazolin-6-yl)amino]phenyl]-3-fluorpyrrolidin-1-sulfonamid;
6-[6-Chlor-2-cyano-3-[[ethyl(methyl)sulfamoyl]amino]anilino]-3-methyl-4-oxochinazolin;
N-[4-Chlor-2-cyano-3-[(3-methyl-4-oxochinazolin-6-yl)amino]phenyl]pyrrolidin-1-sulfonamid;
N-[2-Cyano-4-fluor-3-(3-methyl-4-oxochinazolin-6-yl)oxyphenyl]pyrrolidin-1-sulfonamid;
N-[2-Cyano-4-fluor-3-(3-methyl-4-oxochinazolin-6-yl)oxyphenyl]cyclopentansulfonamid und
6-[2-Cyano-3-(dimethylsulfamoylamino)-6-fluorphenoxy]-3-methyl-4-oxochinazolin; oder ein pharmazeutisch unbedenkliches Salz davon.

13. Verbindung nach Anspruch 12, wobei die Verbindung aus Folgenden ausgewählt ist
6-[2-Cyano-3-[[ethyl(methyl)sulfamoyl]amino]-6-fluorphenoxy]-3-methyl-4-oxochinazolin;
6-[6-Chlor-2-cyano-3-[[ethyl(methyl)sulfamoyl]amino]phenoxy]-3-methyl-4-oxochinazolin;
N-[4-Chlor-2-cyano-3-(3-methyl-4-oxochinazolin-6-yl)oxyphenyl]pyrrolidin-1-sulfonamid;
6-[2-Cyano-3-[[ethyl(methyl)sulfamoyl]amino]phenoxy]-3-methyl-4-oxochinazolin;
N-[2-Cyano-3-(3-methyl-4-oxochinazolin-6-yl)oxyphenyl]pyrrolidin-1-sulfonamid;
N-[2-Cyano-3-(3-methyl-4-oxochinazolin-6-yl)oxyphenyl]cyclopentansulfonamid;
N-[2-Cyano-3-(3-methyl-4-oxochinazolin-6-yl)oxyphenyl]cyclohexansulfonamid;
N-[2-Cyano-3-(3-methyl-4-oxochinazolin-6-yl)oxyphenyl]butan-2-sulfonamid;
6-[2-Cyano-3-[[ethyl(methyl)sulfamoyl]amino]anilino]-3-methyl-4-oxochinazolin;
6-[6-Chlor-2-cyano-3-[[ethyl(methyl)sulfamoyl]amino]anilino]-3-methyl-4-oxochinazolin;
N-[4-Chlor-2-cyano-3-[(3-methyl-4-oxochinazolin-6-yl)amino]phenyl]pyrrolidin-1-sulfonamid;
N-[2-Cyano-4-fluor-3-(3-methyl-4-oxochinazolin-6-yl)oxyphenyl]pyrrolidin-1-sulfonamid;
N-[2-Cyano-4-fluor-3-(3-methyl-4-oxochinazolin-6-yl)oxyphenyl]cyclopentansulfonamid und
6-[2-Cyano-3-(dimethylsulfamoylamino)-6-fluorphenoxy]-3-methyl-4-oxochinazolin; oder ein pharmazeutisch unbedenkliches Salz davon.

14. Verbindung nach einem der Ansprüche 1 bis 13 zur Verwendung als therapeutisch wirksame Substanz.

15. Pharmazeutische Zusammensetzung, umfassend eine Verbindung nach einem der Ansprüche 1 bis 13 und einen therapeutisch inerten Träger.

16. Verbindung nach einem der Ansprüche 1 bis 13 zur Verwendung bei der Behandlung oder Prophylaxe von Schilddrüsenkrebs, Kolorektalkrebs, Hirnkrebs, Melanom oder nicht-kleinzelligem Lungenkrebs.

## Revendications

1. Composé de formule (I) 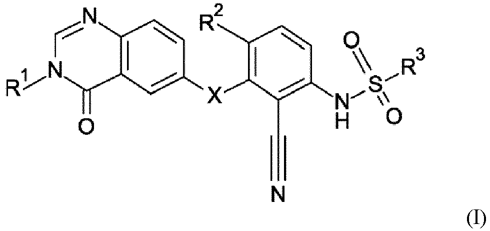 dans lequel
R¹ représente un alkyle en C₁₋₆ ;
X est choisi parmi
i) -NH-, et
ii) -O- ;
R² est choisi parmi
iii) H,
iv) un cyano, et
v) un halogène ;
R³ est choisi parmi
vi) NR⁴R⁵, et
vii) CHR⁶R⁷;
R⁴ est choisi parmi
viii) un alkyle en C₁₋₆,
ix) un cycloalkyle en C₃₋₈, et
x) un cycloalkyle en C₃₋₈-alkyle en C₁₋₆ ;
R⁵ est choisi parmi
xi) un alkyle en C₁₋₆,
xii) un cycloalkyle en C₃₋₈, et
xiii) un cycloalkyle en C₃₋₈-alkyle en C₁₋₆ ;
ou R⁴ et R⁵ conjointement avec l'atome d'azote auquel ils sont liés forment un hétérocycloalkyle éventuellement substitué par R⁸, dans lequel l'hétérocycloalkyle est choisi parmi un pyrrolidinyle et un pipéridinyle ;
R⁶ est choisi parmi
xiv) un alkyle en C₁₋₆,
xv) un cycloalkyle en C₃₋₈, et
xvi) un cycloalkyle en C₃₋₈-alkyle en C₁₋₆ ;
R⁷ est choisi parmi
xvii) un alkyle en C₁₋₆,
xviii) un cycloalkyle en C₃₋₈, et
xix) un cycloalkyle en C₃₋₈-alkyle en C₁₋₆ ;
ou R⁶ et R⁷ conjointement avec l'atome de carbone auquel ils sont liés forment un cycloalkyle en C₃₋₈ éventuellement substitué par R⁸ ;
R⁸ représente un halogène ;
à condition que le N-[2-cyano-4-fluoro-3-(3-méthyl-4-oxo-quinazolin-6-yl)oxy-phényl]-3-fluoro-pyrrolidine-1-sulfonamide soit exclu
ou un sel pharmaceutiquement acceptable de celui-ci.

2. Composé selon la revendication 1, dans lequel
R¹ représente un alkyle en C₁₋₆ ;
X est choisi parmi
i) -NH-, et
ii) -O- ;
R² est choisi parmi
iii) H, et
iv) un halogène ;
R³ est choisi parmi
v) NR⁴R⁵, et
vi) CHR⁶R⁷ ;
R⁴ est choisi parmi
vii) un alkyle en C₁₋₆,
viii) un cycloalkyle en C₃₋₈, et
ix) un cycloalkyle en C₃₋₈-alkyle en C₁₋₆ ;
R⁵ est choisi parmi
x) un alkyle en C₁₋₆,
xi) un cycloalkyle en C₃₋₈, et
xii) un cycloalkyle en C₃₋₈-alkyle en C₁₋₆ ;
ou R⁴ et R⁵ conjointement avec l'atome d'azote auquel ils sont liés forment un hétérocycloalkyle éventuellement substitué par R⁸, dans lequel l'hétérocycloalkyle est choisi parmi un pyrrolidinyle et un pipéridinyle ;
R⁶ est choisi parmi
xiii) un alkyle en C₁₋₆,
xiv) un cycloalkyle en C₃₋₈, et
xv) un cycloalkyle en C₃₋₈-alkyle en C₁₋₆ ;
R⁷ est choisi parmi
xvi) un alkyle en C₁₋₆,
xvii) un cycloalkyle en C₃₋₈, et
xviii) un cycloalkyle en C₃₋₈-alkyle en C₁₋₆ ;
ou R⁶ et R⁷ conjointement avec l'atome de carbone auquel ils sont liés forment un cyclopentyle ou un cycle cyclohexyle éventuellement substitué par R⁸ ;
R⁸ représente un halogène ;
à condition que le (3R)-N-[2-cyano-4-fluoro-3-(3-méthyl-4-oxo-quinazolin-6-yl)oxy-phényl]-3-fluoro-pyrrolidine-1-sulfonamide soit exclu
ou un sel pharmaceutiquement acceptable de celui-ci.

3. Composé selon la revendication 1 ou 2, dans lequel
R¹ représente un méthyle ;
X est choisi parmi
i) -NH-, et
ii) -O- ;
R² est choisi parmi
iii) H,
iv) un chlore, et
v) un fluor ;
R³ est choisi parmi
vi) NR⁴R⁵, et
vii) CHR⁶R⁷;
R⁴ est choisi parmi
viii) un méthyle,
ix) un éthyle,
x) un propyle,
xi) un cyclopropyle, et
xii) un cyclopropylméthyle ;
R⁵ est choisi parmi
xiii) un méthyle,
xiv) un éthyle,
xv) un propyle,
xvi) un cyclopropyle, et
xvii) un cyclopropylméthyle ;
ou R⁴ et R⁵ conjointement avec l'atome d'azote auquel ils sont liés forment un hétérocycloalkyle éventuellement substitué par R⁸, dans lequel l'hétérocycloalkyle est choisi parmi un pyrrolidinyle et un pipéridinyle ;
R⁶ est choisi parmi
xviii) un méthyle,
xix) un éthyle,
xx) un propyle,
xxi) un cyclopropyle, et
xxii) un cyclopropylméthyle ;
R⁷ est choisi parmi
xxiii) un méthyle,
xxiv) un éthyle,
xxv) un propyle,
xxvi) un cyclopropyle, et
xxvii) un cyclopropylméthyle ;
ou R⁶ et R⁷ conjointement avec l'atome de carbone auquel ils sont liés forment un cyclopentyle ou un cycle cyclohexyle éventuellement substitué par R⁸ ;
R⁸ représente un fluor ;
à condition que le (3R)-N-[2-cyano-4-fluoro-3-(3-méthyl-4-oxo-quinazolin-6-yl)oxy-phényl]-3-fluoro-pyrrolidine-1-sulfonamide soit exclu
ou un sel pharmaceutiquement acceptable de celui-ci.

4. Composé selon l'une quelconque des revendications 1 à 3, dans lequel R² est choisi parmi
i) H,
ii) un chlore, et
iii) un fluor.

5. Composé selon l'une quelconque des revendications 1 à 4, dans lequel R⁴ représente un méthyle.

6. Composé selon l'une quelconque des revendications 1 à 5, dans lequel R⁵ représente un éthyle.

7. Composé selon l'une quelconque des revendications 1 à 6, dans lequel R⁴ et R⁵ conjointement avec l'atome d'azote auquel ils sont liés forment un hétérocycloalkyle éventuellement substitué par R⁸, dans lequel l'hétérocycloalkyle est choisi parmi un pyrrolidinyle et un pipéridinyle.

8. Composé selon la revendication 7, dans lequel R⁴ et R⁵ conjointement avec l'atome d'azote auquel ils sont liés forment un hétérocycloalkyle non substitué, dans lequel l'hétérocycloalkyle est un pyrrolidinyle.

9. Composé selon l'une quelconque des revendications 1 à 8, dans lequel R⁶ et R⁷ sont indépendamment choisis parmi
i) un méthyle,
ii) un éthyle,
iii) un propyle,
iv) un cyclopropyle, et
v) un cyclopropylméthyle.

10. Composé selon l'une quelconque des revendications 1 à 8, dans lequel R⁶ et R⁷ conjointement avec l'atome d'azote auquel ils sont liés forment un cyclopentyle ou un cycle cyclohexyle.

11. Composé selon l'une quelconque des revendications 1 à 10, dans lequel R⁸ représente un fluor.

12. Composé selon l'une quelconque des revendications 1 à 11, dans lequel le composé est choisi parmi
la 6-[2-cyano-3-[[éthyl(méthyl)sulfamoyl]amino]-6-fluoro-phénoxy]-3-méthyl-4-oxo-quinazoline ;
la 6-[6-chloro-2-cyano-3-[[éthyl(méthyl)sulfamoyl]amino]phénoxy]-3-méthyl-4-oxo-quinazoline ;
le (3R)-N-[4-chloro-2-cyano-3-(3-méthyl-4-oxo-quinazolin-6-yl)oxy-phényl]-3-fluoro-pyrrolidine-1-sulfonamide ;
le N-[4-chloro-2-cyano-3-(3-méthyl-4-oxo-quinazolin-6-yl)oxy-phényl]pyrrolidine-1-sulfonamide ;
la 6-[2-cyano-3-[[éthyl(méthyl)sulfamoyl]amino]phénoxy]-3-méthyl-4-oxo-quinazoline ;
le N-[2-cyano-3-(3-méthyl-4-oxo-quinazolin-6-yl)oxy-phényl]pyrrolidine-1-sulfonamide ;
le (3R)-N-[2-cyano-3-(3-méthyl-4-oxo-quinazolin-6-yl)oxy-phényl]-3-fluoro-pyrrolidine-1-sulfonamide ;
le N-[2-cyano-3-(3-méthyl-4-oxo-quinazolin-6-yl)oxy-phényl]cyclopentanesulfonamide ;
le N-[2-cyano-3-(3-méthyl-4-oxo-quinazolin-6-yl)oxy-phényl]cyclohexanesulfonamide ;
le N-[2-cyano-3-(3-méthyl-4-oxo-quinazolin-6-yl)oxy-phényl]butane-2-sulfonamide ;
la 6-[2-cyano-3-[[éthyl(méthyl)sulfamoyl]amino]anilino]-3-méthyl-4-oxo-quinazoline ;
le (3R)-N-[2-cyano-3-[(3-méthyl-4-oxo-quinazolin-6-yl)amino]phényl]-3-fluoro-pyrrolidine-1-sulfonamide ;
la 6-[6-chloro-2-cyano-3-[[éthyl(méthyl)sulfamoyl]amino]anilino]-3-méthyl-4-oxo-quinazoline ;
le N-[4-chloro-2-cyano-3-[(3-méthyl-4-oxo-quinazolin-6-yl)amino]phényl]pyrrolidine-1-sulfonamide ;
le N-[2-cyano-4-fluoro-3-(3-méthyl-4-oxo-quinazolin-6-yl)oxy-phényl]pyrrolidine-1-sulfonamide ;
le N-[2-cyano-4-fluoro-3-(3-méthyl-4-oxo-quinazolin-6-yl)oxy-phényl]cyclopentanesulfonamide ; et
la 6-[2-cyano-3-(diméthylsulfamoylamino)-6-fluoro-phénoxy]-3-méthyl-4-oxo-quinazoline ;
ou un sel pharmaceutiquement acceptable de ceux-ci.

13. Composé selon la revendication 12, dans lequel le composé est choisi parmi
la 6-[2-cyano-3-[[éthyl(méthyl)sulfamoyl]amino]-6-fluoro-phénoxy]-3-méthyl-4-oxo-quinazoline ;
la 6-[6-chloro-2-cyano-3-[[éthyl(méthyl)sulfamoyl]amino]phénoxy]-3-méthyl-4-oxo-quinazoline ;
le N-[4-chloro-2-cyano-3-(3-méthyl-4-oxo-quinazolin-6-yl)oxy-phényl]pyrrolidine-1-sulfonamide ;
la 6-[2-cyano-3-[[éthyl(méthyl)sulfamoyl]amino]phénoxy]-3-méthyl-4-oxo-quinazoline ;
le N-[2-cyano-3-(3-méthyl-4-oxo-quinazolin-6-yl)oxy-phényl]pyrrolidine-1-sulfonamide ;
le N-[2-cyano-3-(3-méthyl-4-oxo-quinazolin-6-yl)oxy-phényl]cyclopentanesulfonamide ;
le N-[2-cyano-3-(3-méthyl-4-oxo-quinazolin-6-yl)oxy-phényl]cyclohexanesulfonamide ;
le N-[2-cyano-3-(3-méthyl-4-oxo-quinazolin-6-yl)oxy-phényl]butane-2-sulfonamide ;
la 6-[2-cyano-3-[[éthyl(méthyl)sulfamoyl]amino]anilino]-3-méthyl-4-oxo-quinazoline ;
la 6-[6-chloro-2-cyano-3-[[éthyl(méthyl)sulfamoyl]amino]anilino]-3-méthyl-4-oxo-quinazoline ;
le N-[4-chloro-2-cyano-3-[(3-méthyl-4-oxo-quinazolin-6-yl)amino]phényl]pyrrolidine-1-sulfonamide ;
le N-[2-cyano-4-fluoro-3-(3-méthyl-4-oxo-quinazolin-6-yl)oxy-phényl]pyrrolidine-1-sulfonamide ;
le N-[2-cyano-4-fluoro-3-(3-méthyl-4-oxo-quinazolin-6-yl)oxy-phényl]cyclopentanesulfonamide ; et
la 6-[2-cyano-3-(diméthylsulfamoylamino)-6-fluoro-phénoxy]-3-méthyl-4-oxo-quinazoline ;
ou un sel pharmaceutiquement acceptable de ceux-ci.

14. Composé selon l'une quelconque des revendications 1 à 13 pour une utilisation comme substance thérapeutiquement active.

15. Composition pharmaceutique comprenant un composé selon l'une quelconque des revendications 1 à 13 et un véhicule thérapeutiquement inerte.

16. Composé selon l'une quelconque des revendications 1 à 13 pour une utilisation dans le traitement ou la prophylaxie du cancer de la thyroïde, du cancer colorectal, du cancer du cerveau, du mélanome ou du cancer du poumon non à petites cellules.
